(19) Europäisches Patentamt
European Patent Office
Office européen des brevets

(11) Veröffentlichungsnummer: **0 330 057 A2**

# (12) EUROPÄISCHE PATENTANMELDUNG

(21) Anmeldenummer: **89102536.3**

(22) Anmeldetag: **15.02.89**

(51) Int. Cl.4: **C07D 239/26 , C07D 405/06 , A61K 31/505**

(30) Priorität: **25.02.88 DE 3805913**
**24.11.88 IT 2272188**

(43) Veröffentlichungstag der Anmeldung:
**30.08.89 Patentblatt 89/35**

(84) Benannte Vertragsstaaten:
**AT BE CH DE ES FR GB GR IT LI LU NL SE**

(71) Anmelder: **BAYER AG**
**D-5090 Leverkusen 1 Bayerwerk(DE)**

(72) Erfinder: **Fey, Peter, Dr.**
**Ursulastrasse 14**
**D-5600 Wuppertal 1(DE)**
Erfinder: **Angerbauer, Rolf, Dr.**
**Sterntalerweg 29**
**D-5600 Wuppertal 1(DE)**
Erfinder: **Hübsch, Walter, Dr.**
**Am Eckbusch 43/50**
**D-5600 Wuppertal 1(DE)**
Erfinder: **Bischoff, Hilmar, Dr.**
**Am Rohm 78**
**D-5600 Wuppertal 2(DE)**
Erfinder: **Petzinna, Dieter, Dr.**
**Peter-Berten-Strasse**
**D-4000 Düsseldorf 21(DE)**
Erfinder: **Schmidt, Delf, Dr.**
**Am Eckbusch 55b**
**D-5600 Wuppertal 1(DE)**
Erfinder: **Thomas, Günter, Dr.**
**Via Campogallo 21/14**
**I-2002 Arese(IT)**

## (54) Substituierte Pyrimidine.

(57) Substituierte Pyrimidine können durch Reduktion entsprechender Ketone und gegebenenfalls nachfolgender Verseifung, Cyclisierung, Hydrierung und Isomerentrennung hergestellt werden. Die neuen Verbindungen können als Wirkstoffe in Arzneimittel verwendet werden.

EP 0 330 057 A2

## Substituierte Pyrimidine

Die Erfindung betrifft substituierte Pyrimidine, Zwischenverbindungen zu ihrer Herstellung, ihre Herstellung und ihre Verwendung in Arzneimitteln.

Es ist bekannt, daß aus Pilzkulturen isolierte Lactonderivate Inhibitoren der 3-Hydroxy-3-methyl-glutaryl Coenzym A Reduktase (HMG-CoA-Reduktase) sind [EP-A 22 478; US 4 231 938]. Darüberhinaus sind auch bestimmte Indolderivate bzw. Pyrazolderivate Inhibitoren der HMG-CoA-Reduktase [EP-A 1 114 027; US-Patent 4 613 610].

Es wurden substituierte Pyrimidine der allgemeinen Formel (I),

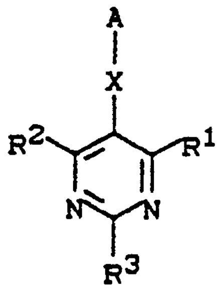

(I)

in welcher

$R^1$ - für Cycloalkyl steht, oder

- für Alkyl steht, das substituiert sein kann durch Halogen, Cyano, Alkoxy, Alkylthio, Alkylsulfonyl, Trifluormethyl, Trifluormethoxy, Trifluormethylthio, Trifluormethylsulfonyl, Alkoxycarbonyl, Acyl oder durch eine Gruppe der Formel $-NR^4R^5$, worin

$R^4$, $R^5$ - gleich oder verschieden sind und Alkyl, Aryl, Aralkyl, Acyl, Alkylsulfonyl oder Arylsulfonyl bedeuten,

oder durch Carbamoyl, Dialkylcarbamoyl, Sulfamoyl, Dialkylsulfamoyl, Heteroaryl, Aryl, Aryloxy, Arylthio, Arylsulfonyl, Aralkoxy, Aralkylthio oder Aralkylsulfonyl, wobei die Heteroaryl- und Arylreste der letztgenannten Substituenten bis zu 3-fach gleich oder verschieden durch Halogen, Cyano, Trifluormethyl, Trifluormethoxy, Alkyl, Alkoxy, Alkylthio oder Alkylsulfonyl substituiert sein können,

$R^2$ - für Heteroaryl steht, das bis zu 3-fach gleich oder verschieden durch Halogen, Alkyl, Alkoxy, Alkylthio, Alkylsulfonyl, Aryl, Aryloxy, Arylthio, Arylsulfonyl, Trifluormethyl, Trifluormethoxy, Trifluormethylthio, Alkoxycarbonyl oder durch eine Gruppe der Formel $-NR^4R^5$ substitutiert sein kann, worin $R^4$, $R^5$ die oben angegebene Bedeutung haben, oder

$R^2$ - für Aryl steht, das bis zu 5-fach gleich oder verschieden substituiert sein kann durch Alkyl, Alkoxy, Alkylthio, Alkylsulfonyl, Aryl, Aryloxy, Arylthio, Arylsulfonyl, Aralkyl, Aralkoxy, Aralkylthio, Aralkylsulfonyl, Halogen, Cyano, Nitro, Trifluormethyl, Trifluormethoxy, Trifluormethylthio, Alkoxycarbonyl, Sulfamoyl, Dialkylsulfamoyl, Carbamoyl, Dialkylcarbamoyl oder durch eine Gruppe der Formel $-NR^4R^5$, worin $R^4$ und $R^5$ die oben angegebene Bedeutung haben,

$R^3$ - für Wasserstoff oder

- für Cycloalkyl steht, oder

- für Alkyl steht, das substituiert sein kann durch Halogen, Cyano, Alkoxy, Alkylthio, Alkylsulfonyl, Trifluormethyl, Trifluormethoxy, Trifluormethylthio, Trifluormethylsulfonyl, Alkoxycarbonyl, Acyl oder durch eine Gruppe der Formel $-NR^4R^5$, worin $R^4$, $R^5$ die oben angegebene Bedeutung haben,

oder durch Carbamoyl, Dialkylcarbamoyl, Sulfamoyl, Dialkylsulfamoyl, Heteroaryl, Aryl, Aryloxy, Arylthio, Arylsulfonyl, Aralkoxy, Aralkylthio oder Aralkylsulfonyl, wobei die Heteroaryl- und Arylreste der letztgenannten Substituenten bis zu 3-fach gleich oder verschieden durch Halogen, Cyano, Trifluormethyl, Trifluormethoxy, Alkyl, Alkoxy, Alkylthio oder Alkylsulfonyl substituiert sein können, oder

$R^3$ - für Heteroaryl steht, das bis zu 3-fach gleich oder verschieden durch Halogen, Alkyl, Alkoxy, Alkylthio, Alkylsulfonyl, Aryl, Aryloxy, Arylthio, Arylsulfonyl, Trifluormethyl, Trifluormethoxy, Trifluormethylthio, Alkoxycarbonyl oder durch eine Gruppe der Formel $-NR^4R^5$ substituiert sein kann, worin $R^4$ und $R^5$ die oben angegebene Bedeutung haben, oder

$R^3$ - für Aryl steht, das bis zu 5-fach gleich oder verschieden substituiert sein kann durch Alkyl, Alkoxy, Alkylthio, Alkylsulfonyl, Aryl, Aryloxy, Arylthio, Arylsulfonyl, Aralkyl, Aralkoxy, Aralkylthio, Aralkylsulfonyl, Halogen, Cyano, Nitro, Trifluormethyl, Trifluormethoxy, Trifluormethylthio, Alkoxycarbonyl, Sulfamoyl, Dialkylsulfamoyl, Carbamoyl, Dialkylcarbamoyl oder durch eine Gruppe der Formel $-NR^4R^5$, worin $R^4$ und $R^5$ die oben angegebene Bedeutung haben, oder

$R^3$ - für Alkoxy, Aryloxy, Aralkoxy, Alkylthio, Arylthio, Aralkylthio oder für eine Gruppe der Formel $-NR^4R^5$ steht, worin $R^4$ und $R^5$ die oben angegebene Bedeutung haben,

X - für eine Gruppe der Formel $-CH_2-CH_2-$ oder $-CH=CH-$steht und

A - für eine Gruppe der Formel

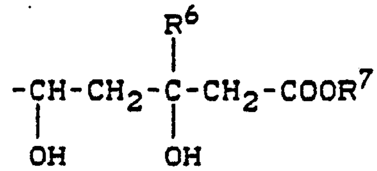

oder

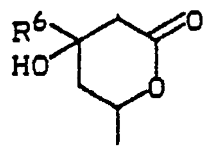

steht, worin

$R^6$ - Wasserstoff oder Alkyl bedeutet und

$R^7$ - Wasserstoff,

- einen Alkyl-, Aryl- oder Aralkylrest oder
- ein Kation bedeutet

gefunden.

Überraschenderweise zeigen die erfindungsgemäßen substituierten Pyrimidine eine gute inhibitorische Wirkung auf die HMG-CoA Reduktase (3-Hydroxy-3-methyl-glutaryl Coenzym A Reduktase).

Cycloalkyl steht im allgemeinen für eine cyclischen Kohlenwasserstoffrest mit 3 bis 8 Kohlenstoffatomen. Bevorzugt ist der Cyclopropyl-, Cyclopentyl- und der Cyclohexylring. Beispielsweise seien Cyclopropyl, Cyclopentyl, Cyclohexyl, Cycloheptyl und Cyclooctyl genannt.

Alkyl steht im allgemeinen für einen geradkettigen oder verzweigten Kohlenwasserstoffrest mit 1 bis 12 Kohlenstoffatomen. Bevorzugt wird Niederalkyl mit 1 bis etwa 6 Kohlenstoffatomen. Beispielsweise seien Methyl, Ethyl, Propyl, Isopropyl, Butyl, Isobutyl, Pentyl, Isopentyl, Hexyl, Isohexyl, Heptyl, Isoheptyl, Octyl und Isooctyl genannt.

Alkoxy steht im allgemeinen für einen über ein Sauerstoffatom gebundenen geradkettigen oder verzweigten Kohlenwasserstoffrest mit 1 bis 12 Kohlenstoffatomen. Bevorzugt ist Niederalkoxy mit 1 bis etwa 6 Kohlenstoffatomen. Besonders bevorzugt ist ein Alkoxyrest mit 1 bis 4 Kohlenstoffatomen. Beispielsweise seien Methoxy, Ethoxy, Propoxy, Isopropoxy, Butoxy, Isobutoxy, Pentoxy, Isopentoxy, Hexoxy, Isohexoxy, Heptoxy, Isoheptoxy, Octoxy oder Isooctoxy genannt.

Alkylthio steht im allgemeinen für einen über ein Schwefelatom gebundenen geradkettigen oder verzweigten Kohlenwasserstoffrest mit 1 bis 12 Kohlenstoffatomen. Bevorzugt ist Niederalkylthio mit 1 bis etwa 6 Kohlenstoffatomen. Besonders bevorzugt ist ein Alkylthiorest mit 1 bis 4 Kohlenstoffatomen. Beispielsweise seien Methylthio, Ethylthio, Propylthio, Isopropylthio, Butylthio, Isobutylthio, Pentylthio, Isopentylthio, Hexylthio, Isohexylthio, Heptylthio, Isoheptylthio, Octylthio oder Isooctylthio genannt.

Alkylsulfonyl steht im allgemeinen für einen geradkettigen oder verzweigten Kohlenwasserstoffrest mit 1 bis 12 Kohlenstoffatomen, der über eine $SO_2$-Gruppe gebunden ist. Bevorzugt ist Niederalkylsulfonyl mit 1 bis etwa 6 Kohlenstoffatomen. Beispielsweise seien genannt: Methylsulfonyl, Ethylsulfonyl, Propylsulfonyl, Isopropylsulfonyl, Butylsulfonyl, Isobutylsulfonyl, Pentylsulfonyl, Isopentylsulfonyl, Hexylsulfonyl, Isohexylsulfonyl.

Aryl steht im allgemeinen für einen aromatischen Rest mit 6 bis etwa 12 Kohlenstoffatomen. Bevorzugte Arylreste sind Phenyl, Naphthyl und Biphenyl.

Aryloxy steht im allgemeinen für einen aromatischen Rest mit 6 bis etwa 12 Kohlenstoffatomen, der über ein Sauerstoffatom gebunden ist. Bevorzugte Aryloxyreste sind Phenoxy oder Naphthyloxy.

Arylthio steht im allgemeinen für einen aromatischen Rest mit 6 bis etwa 12 Kohlenstoffatomen, der über ein Schwefelatom gebunden ist. Bevorzugte Arylthioreste sind Phenylthio oder Naphthylthio.

Arylsulfonyl steht im allgemeinen für einen aromatischen Rest mit 6 bis etwa 12 Kohlenstoffatomen, der über eine $SO_2$-Gruppe gebunden ist. Beispielsweise seien genannt: Phenylsulfonyl, Naphthylsulfonyl und Biphenylsulfonyl.

Aralkyl steht im allgemeinen für einen über eine Alkylenkette gebundenen Arylrest mit 7 bis 14 Kohlenstoffatomen. Bevorzugt werden Aralkylreste mit 1 bis 6 Kohlenstoffatomen im aliphatischen Teil und 6 bis 12 Kohlenstoffatome im aromatischen Teil. Beispielsweise seien folgende Aralkylreste genannt: Benzyl, Naphthylmethyl, Phenethyl und Phenylpropyl.

Aralkoxy steht im allgemeinen für einen Aralkylrest mit 7 bis 14 Kohlenstoffatomen, wobei die

Alkylenkette über ein Sauerstoffatom gebunden ist. Bevorzugt werden Aralkoxyreste mit 1 bis 6 Kohlenstoffatomen im aliphatischen Teil und 6 bis 12 Kohlenstoffatomen im aromatischen Teil. Beispielsweise seien folgende Aralkoxyreste genannt: Benzyloxy, Naphthylmethoxy, Phenethoxy und Phenylpropoxy.

Aralkylthio steht im allgemeinen für einen Aralkylrest mit 7 bis etwa 14 Kohlenstoffatomen wobei die Alkylkette über ein Schwefelatom gebunden ist. Bevorzugt werden Aralkylthioreste mit 1 bis 6 Kohlenstoffatomen im aliphatischen Teil und 6 bis 12 Kohlenstoffatomen im aromatischen Teil. Beispielsweise seien folgende Aralkylthioreste genannt: Benzylthio, Naphthylmethylthio, Phenethylthio und Phenylpropylthio.

Aralkylsulfonyl steht im allgemeinen für einen Aralkylrest mit 7 bis etwa 14 Kohlenstoffatomen, wobei die Alkylreste über eine $SO_2$-Kette gebunden ist. Bevorzugt werden Aralkylsulfonylreste mit 1 bis 6 Kohlenstoffatomen im aliphatischen Teil und 6 bis 12 Kohlenstoffatomen im aromatischen Teil. Beispielsweise seien folgende Aralkylsulfonylreste genannt: Benzylsulfonyl, Naphthylmethylsulfonyl, Phenethylsulfonyl und Phenylpropylsulfonyl.

Alkoxycarbonyl kann beispielsweise durch die Formel

$$-\underset{\underset{O}{\|}}{C}-OAlkyl$$

dargestellt werden. Alkyl steht hierbei für einen geradkettigen oder verzweigten Kohlenwasserstoffrest mit 1 bis 12 Kohlenstoffatomen. Bevorzugt wird Niederalkoxycarbonyl mit 1 bis etwa 6 Kohlenstoffatomen im Alkylteil. Insbesondere bevorzugt wird ein Alkoxycarbonyl mit 1 bis 4 Kohlenstoffatomen im Alkylteil. Beispielsweise seien die folgenden Alkoxycarbonylreste genannt: Methoxycarbonyl, Ethoxycarbonyl, Propoxycarbonyl, Isopropoxycarbonyl, Butoxycarbonyl oder Isobutoxycarbonyl.

Acyl steht im allgemeinen für Phenyl oder geradkettiges oder verzweigtes Niederalkyl mit 1 bis etwa 6 Kohlenstoffatomen, die über eine Carbonylgruppe gebunden sind. Bevorzugt sind Phenyl und Alkylreste mit bis zu 4 Kohlenstoffatomen. Beispielsweise seien genannt: Benzoyl, Acetyl, Ethylcarbonyl, Propylcarbonyl, Isopropylcarbonyl, Butylcarbonyl und Isobutylcarbonyl.

Halogen steht im allgemeinen für Fluor, Chlor, Brom oder Iod, bevorzugt für Fluor, Chlor oder Brom. Besonders bevorzugt steht Halogen für Fluor oder Chlor.

Heteroaryl im Rahmen der oben angebenen Definition steht im allgemeinen für einen 5- bis 6-gliedrigen aromatischen Ring, der als Heteroatome Sauerstoff, Schwefel und/oder Stickstoff enthalten kann und an den weitere aromatische Ringe ankondensiert sein können. Bevorzugt sind 5- und 6-gliedrige aromatische Ringe, die einen Sauerstoff, ein Schwefel und/oder bis zu 2 Stickstoffatome enthalten und die gegebenenfalls benzokondensiert sind. Als besonders bevorzugte Heteroarylreste seien genannt: Thienyl, Furyl, Pyrolyl, Pyrazolyl, Pyridyl, Pyrimidyl, Pyrazinyl, Pyridazinyl, Chinolyl, Isochinolyl, Chinazolyl, Chinoxalyl, Phthalazinyl, Cinnolyl, Thiazolyl, Benzothiazolyl, Isothiazolyl, Oxazolyl, Benzoxazolyl, Isoxazolyl, Imidazolyl, Benzimidazolyl, Pyrazolyl, Indolyl, und Isoindolyl.

Falls $R^7$ für einen Alkyl-, Aryl- oder Aralkylrest steht, bildet sich eine Estergruppe.

Bevorzugt werden physiologisch verträgliche Ester, die in vivo leicht zu einer freien Carboxylgruppe und einem entsprechenden physiologisch verträglichen Alkohol hydrolysiert werden. Hierzu gehören beispielsweise Alkylester ($C_1$ bis $C_6$-Aryl ($C_6$ bis $C_{12}$), und Aralkylester ($C_7$ bis $C_{10}$), bevorzugt Niederalkylester sowie Benzylester. Insbesondere bevorzugt werden Methylester, Ethylester, Propylester, Benzylester.

Steht $R^7$ für ein Kation so, ist bevorzugt ein physiologisch verträgliches Metall-oder Ammoniumkation gemeint. Bevorzugt sind hierbei Alkali-bzw. Erdalkalikationen wie beispielsweise Natrium-, Kalium-, Magnesium- oder Calciumkationen, sowie Aluminium-oder Ammoniumkationen, sowie nicht-toxische substituierte Ammoniumkationen aus Aminen wie Diniederalkylamine ($C_9$ bis etwa $C_6$), Triniederalkylamine ($C_1$ bis etwa $C_6$), Dibenzylamin, N,N′-Dibenzylethylendiamin, N-Benzyl-β-phenylethylamin, N-Methylmorpholin oder N-Ethylmorpholin, Dihydroabiethylamin, N,N′-Bis-dihydroabiethylethylendiamin, N-Niederalkylpiperidin und andere Amine, die zur Bildung von Salzen verwendet werden können.

Bevorzugt werden substituierte Pyrimidine der Formel (I), in welcher

$R^1$ - für Cyclopropyl, Cyclopentyl oder Cyclohexyl steht, oder

- für Niederalkyl steht, das substituiert sein kann durch Fluor, Chlor, Brom, Cyano, Niederalkoxy, Niederalkylthio, Niederalkylsulfonyl, Trifluormethyl, Trifluormethoxy, Trifluormethylsulfonyl, Niederalkoxycarbonyl, Benzoyl, Niederalkylcarbonyl, durch eine Gruppe der Formel $-NR^4R^5$, worin

$R^4$ und $R^5$ gleich oder verschieden sind und Niederalkyl, Phenyl, Benzyl, Acetyl, Benzoyl, Phenylsulfonyl oder Niederalkylsulfonyl bedeuten,

oder durch Pyridyl, Pyrimidyl, Pyrazinyl, Pyridazinyl, Chinolyl, Isochinolyl, Pyrryl, Indolyl, Thienyl, Furyl, Imidazolyl, Oxazolyl, Thiazolyl, Phenyl, Phenoxy, Phenylthio, Phenylsulfonyl, Benzyloxy, Benzylthio, Benzylsulfonyl, Phenylethoxy, Phenylethylthio oder Phenylethylsulfonyl, wobei die ge nannten Heteroaryl- und Arylreste bis zu 2-fach gleich oder verschieden durch Fluor, Chlor, Brom, Niederalkyl, Niederalkoxy, Trifluormethyl, oder Trifluormethoxy substituiert sein können,

$R^2$ - für Thienyl, Furyl, Thiazolyl, Isothiazolyl, Oxazolyl, Isoxazolyl, Pyridyl, Pyrimidyl, Pyrazinyl, Pyridazinyl, Indolyl, Isoindolyl, Chinolyl, Isochinolyl, Phthalazinyl, Chinoxalinyl, Chinazolinyl, Cinnolinyl, Benzothiazolyl, Benzoxazolyl oder Benzimidazolyl steht, das bis zu 2-fach gleich oder verschieden substituiert sein kann durch Fluor, Chlor, Brom, Niederalkyl, Niederalkoxy, Phenyl, Phenoxy, Trifluormethyl, Trifluormethoxy oder Niederalkoxycarbonyl, oder

- für Phenyl oder Naphthyl steht, die bis zu 4-fach gleich oder verschieden substituiert sein kann durch Niederalkyl, Niederalkoxy, Niederalkylthio, Niederalkylsulfonyl, Phenyl, Phenyloxy, Phenylthio, Phenylsulfonyl, Benzyl, Benzyloxy, Benzylthio, Benzylsulfonyl, Phenethyl, Phenylethoxy, Phenylethylthio, Phenylethylsulfonyl, Fluor, Chlor, Brom, Cyano, Trifluormethyl, Trifluormethoxy, Trifluormethylthio, Niederalkoxycarbonyl oder durch eine Gruppe der Formel $-NR^4R^5$, wobei $R^4$ und $R^5$ die oben angegebene Bedeutung haben,

$R^3$ - für Wasserstoff steht oder

- für Cyclopropyl, Cyclopentyl oder Cyclohexyl steht oder

- für Niederalkyl steht, das substituiert sein kann durch Fluor, Chlor, Brom, Cyano, Niederalkoxy, Niederalkylthio, Niederalkylsulfonyl, Trifluormethyl, Trifluormethoxy, Trifluormethylsulfonyl, Niederalkoxycarbonyl, Benzoyl, Niederalkylcarbonyl, durch eine Gruppe der Formel $-NR^4R^5$, worin $R^4$, $R^5$ die oben angegebene Bedeutung haben,

oder durch Pyridyl, Pyrimidyl, Pyrazinyl, Pyridazinyl, Chinolyl, Isochinolyl, Pyrrolyl, Indolyl, Thienyl, Furyl, Imidazolyl, Oxazolyl, Thiazolyl, Phenyl, Phenoxy, Phenylthio, Phenylsulfonyl, Benzyloxy, Benzylthio, Benzylsulfonyl, Phenylethoxy, Phenylethylthio oder Phenylethylsulfonyl, wobei die genannten Heteroaryl- und Arylreste bis zu 2-fach gleich oder verschieden durch Fluor, Chlor, Brom, Niederalkyl, Niederalkoxy, Trifluormethyl, oder Trifluormethoxy substituiert sein können, oder

$R^3$ - für Thienyl, Furyl, Thiazolyl, Isothiazolyl, Oxazolyl, Isoxazolyl, Pyridyl, Pyrimidyl, Pyrazinyl, Pyridazinyl, Indolyl, Isoindolyl, Chinolyl, Isochinolyl, Phthalazinyl, Chinoxalinyl, Chinazolinyl, Cinnolinyl, Benzothiazolyl, Benzoxazolyl oder Benzimidazolyl steht, das bis zu 2-fach gleich oder verschieden substituiert sein können durch Fluor, Chlor, Brom, Niederalkyl, Niederalkoxy, Phenyl, Phenoxy, Trifluormethyl, Trifluormethoxy oder Niederalkoxycarbonyl, oder

$R^3$ - für Phenyl oder Naphthyl steht, das bis zu 4-fach gleich oder verschieden substituiert sein kann durch Niederalkyl, Niederalkoxy, Niederalkylthio, Niederalkylsulfonyl, Phenol, Phenyloxy, Phenylthio, Phenylsulfonyl, Benzyl, Benzyloxy, Benzylthio, Benzylsulfonyl, Phenethyl, Phenylethoxy, Phenylethylthio, Phenylethylsulfonyl, Fluor, Chlor, Brom, Cyano, Trifluormethyl, Trifluormethoxy, Trifluormethylthio, Niederalkoxycarbonyl oder durch eine Gruppe der Formel $-NR^4R^5$, wobei $R^4$ und $R^5$ die oben angegebene Bedeutung haben, oder

$R^3$ - für Alkoxy, Aryloxy, Aralkoxy, Alkylthio, Arylthio, Aralkylthio oder für eine Gruppe der Formel $-NR^4R^5$ steht, worin $R^4$ und $R^5$ die oben angegebene Bedeutung haben,

X - für eine Gruppe der Formel $-CH_2-CH_2-$ oder $-CH=CH-$steht und

A - für eine Gruppe der Formel

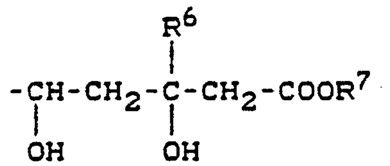

oder

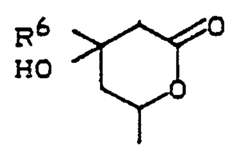

steht, worin

$R^6$ - Wasserstoff oder Niederalkyl bedeutet, und

$R^7$ - Alkyl ($C_1$ bis $C_6$), Aryl ($C_6$ bis $C_{12}$) oder Aralkyl ($C_7$ bis $C_{10}$) bedeutet, oder

- ein physiologisch verträgliches Kation bedeutet.

Besonders bevorzugt sind Verbindungen der allgemeinen Formel (I), in welcher

$R^1$ - für Cyclopropyl, Cyclopentyl oder Cyclohexyl steht, oder

- für Methyl, Ethyl, Propyl, Isopropyl, Butyl, sec.-Butyl oder tert.Butyl steht, die substituiert sein können durch Fluor, Chlor, Brom, Cyano, Methoxy, Ethoxy, Propoxy, Isopropoxy, Butoxy, sec.Butoxy, tert.Butoxy, Methylthio, Ethylthio, Propylthio, Isopropylthio, Methylsulfonyl, Ethylsulfonyl, Propylsulfonyl, Isopropylsulfonyl, Trifluormethyl, Trifluormethoxy, Methoxycarbonyl, Ethoxycarbonyl, Butoxycarbonyl, Isobutoxycarbonyl, tert.-Butoxycarbonyl, Benzoyl, Acetyl, Pyridyl, Pyrimidyl, Thienyl, Furyl, Phenyl, Phenoxy, Phenylthio, Phenylsulfonyl, Benzyloxy, Benzylthio oder Benzylsulfonyl,

$R^2$ - für Pyridyl, Pyrimidyl, Chinolyl oder Isochinolyl steht, das durch Fluor, Chlor, Methyl, Methoxy oder

Trifluormethyl substituiert sein kann, oder
- für Phenyl steht, das bis zu 3-fach gleich oder verschieden substituiert sein kann durch Methyl, Ethyl, Propyl, Isopropyl, Butyl, Isobutyl, tert.-Butyl, Methoxy, Ethoxy, Propoxy, Isopropoxy, Butoxy, Isobutoxy, tert.Butoxy, Methylthio, Ethylthio, Propylthio, Isopropylthio, Methylsulfonyl, Ethylsulfonyl, Propylsulfonyl, Isopropylsulfonyl, Phenyl, Phenoxy, Benzyl, Benzyloxy, Fluor, Chlor, Brom, Cyano, Trifluormethyl, Trifluormethoxy, Methoxycarbonyl, Ethoxycarbonyl, Propoxycarbonyl, Isopropoxycarbonyl, Butoxycarbonyl, Isobutoxycarbonyl oder tert.-Butoxycarbonyl,
$R^3$ - für Wasserstoff, Cyclopropyl, Cyclopentyl oder Cyclohexyl steht, oder
- für Methyl, Ethyl, Propyl, Isopropyl, Butyl, Isobutyl, tert.Butyl, Pentyl, Isopentyl, Hexyl oder Isohexyl steht, das substituiert sein kann durch Fluor, Chlor, Brom, Cyano, Methoxy, Ethoxy, Propoxy, Isopropoxy, Butoxy, Isobutoxy, tert.-Butoxy, Methylthio, Ethylthio, Propylthio, Isopropylthio, Butylthio, Isobutylthio, tert.Butylthio, Methylsulfonyl, Ethylsulfonyl, Propylsulfonyl, Isopropylsulfonyl, Butylsulfonyl, Isobutylsulfonyl, tert.-Butylsulfonyl, Trifluormethyl, Trifluormethoxy, Methoxycarbonyl, Ethoxycarbonyl, Propoxycarbonyl, Isopropoxycarbonyl, Butoxycarbonyl, Isobutoxycarbonyl, tert.-Butoxycarbonyl, Benzoyl, Acetyl, Ethylcarbonyl, oder durch eine Gruppe $-NR^4R^5$, wobei
$R^4$ und $R^5$ gleich oder verschieden sind und Methyl, Ethyl, Propyl, Isopropyl, Butyl, Isobutyl, tert.Butyl, Phenyl, Benzyl, Acetyl, Methylsulfonyl, Ethylsulfonyl, Propylsulfonyl, Isopropylsulfonyl oder Phenylsulfonyl bedeuten,
oder durch Pyridyl, Pyrimidyl, Pyrazinyl, Pyridazinyl, Chinolyl, Isochinolyl, Thienyl, Furyl, Phenyl, Phenoxy, Phenylthio, Phenylsulfonyl, Benzyloxy, Benzylthio oder Benzylsulfonyl, wobei die genannten Heteroaryl- und Arylreste durch Fluor, Chlor, Methyl, Ethyl, Propyl, Isopropyl, Isobutyl, tert. Butyl, Methoxy, Ethoxy, Propoxy, Isopropoxy, Butoxy, Isobutoxy, tert.-Butoxy, Trifluormethyl oder Trifluormethoxy substituiert sein können, oder
- für Thienyl, Furyl, Pyridyl, Pyrimidyl, Pyrazinyl, Pyridazinyl, Oxazolyl, Isooxazolyl, Imidazolyl, Pyrazolyl, Thiazolyl, Isothiazolyl, Chinolyl, Isochinolyl, Benzoxazolyl, Benzimidazolyl oder Benzthiazolyl steht, wobei die genannten Reste durch Fluor, Chlor, Methyl, Ethyl, Propyl, Isopropyl, Butyl, Isobutyl, tert.Butyl, Methoxy, Ethoxy, Propoxy, Isopropoxy, Butoxy, Isobutoxy, tert.Butoxy, Phenyl, Phenoxy, Trifluormethyl, Trifluormethoxy, Methoxycarbonyl, Ethoxycarbonyl, Isopropoxycarbonyl, Propoxycarbonyl, Butoxycarbonyl, Isobutoxycarbonyl oder tert.Butoxycarbonyl substituiert sein können, oder
- für Phenyl steht, das bis zu 3-fach gleich oder verschieden durch Methyl, Ethyl, Propyl, Isopropyl, Butyl, Isobutyl, tert.Butyl, Pentyl, Isopentyl, Hexyl, Isohexyl, Methoxy, Ethoxy, Propoxy, Isopropoxy, Butoxy, Isobutoxy, tert.Butoxy, Methylthio, Ethylthio, Propylthio, Isopropylthio, Butylthio, Isobutylthio, tert.Butylthio, Methylsulfonyl, Ethylsulfonyl, Propylsulfonyl, Isopropylsulfonyl, Butylsulfonyl, Isobutylsulfonyl, tert.Butylsulfonyl, Phenyl, Phenoxy, Phenylthio, Phenylsulfonyl, Benzyl, Benzyloxy, Benzylthio, Benzylsulfonyl, Fluor, Chlor, Brom, Cyano, Trifluormethyl, Trifluormethoxy, Trifluormethylthio, Methoxycarbonyl, Ethoxycarbonyl, Propoxycarbonyl, Isopropoxycarbonyl, Butoxycarbonyl, Isobutoxycarbonyl, tert.Butoxycarbonyl oder durch eine Gruppe $-NR^4R^5$ substituiert sein kann, wobei $R^4$ und $R^5$ die oben angegebene Bedeutung haben, oder
- für Alkoxy, Aryloxy, Aralkoxy, Alkylthio, Arylthio, Aralkylthio oder für eine Gruppe der Formel $-NR^4R^5$ steht, worin $R^4$ und $R^5$ die oben angegebene Bedeutung haben,
X - für eine Gruppe der Formel $-CH_2-CH_2-$ oder $-CH=CH-$steht und
A - für eine Gruppe der Formel

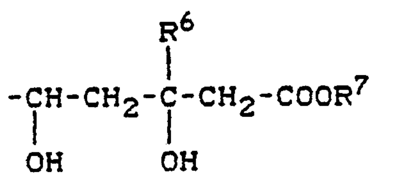 oder 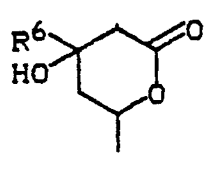

steht, worin
$R^6$ - Wasserstoff, Methyl, Ethyl, Propyl, Isopropyl, Butyl, Isobutyl oder tert.Butyl bedeutet, und
$R^7$ - Wasserstoff, Methyl, Ethyl, Propyl, Isopropyl, Butyl, Isobutyl, tert.Butyl oder Benzyl bedeutet, oder ein Natrium-, Kalium-, Calcium -, oder Magnesium- oder Ammoniumion bedeutet.

Die erfindungsgemäßen substituierten Pyrimidine der allgemeinen Formel (I) haben mehrere asymmetrische Kohlenstoffatome und können daher in verschiedenen stereochemischen Formen existieren. Sowohl die einzelnen Isomeren als auch deren Mischungen sind Gegenstand der Erfindung.

Je nach der Bedeutung der Gruppe X bzw. des Restes A ergeben sich unterschiedliche Stereoisomere,

die im folgenden näher erläutert werden sollen:

a) Steht die Gruppe -X- für eine Gruppe der Formel -CH=CH- , so können die erfindungsgemäßen Verbindungen in zwei stereoisomeren Formen existieren, die an der Doppelbindung E-konfiguriert (II) oder Z-konfiguriert (III) sein können:

(II) E-Form

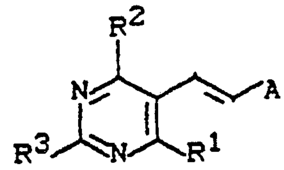

(III) Z-Form

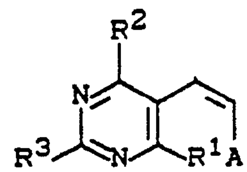

Bevorzugt sind diejenigen Verbindungen der allgemeinen Formel (I) die E-konfiguriert sind (II).

b) Steht der Rest -A- für eine Gruppe der Formel

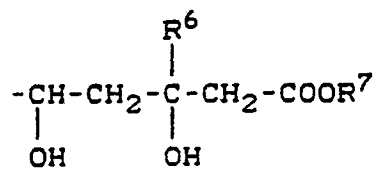

so besitzen die Verbindungen der allgemeinen Formel (I) mindestens zwei asymmetrische Kohlenstoffatome, nämlich die beiden Kohlenstoffatome an denen die Hydroxygruppen gebunden sind. Je nach der relativen Stellung dieser Hydroxygruppen zueinander, können die erfindungsgemäßen Verbindungen in der erythro-Konfiguration (IV) oder in der threo-Konfiguration (V) vorliegen.

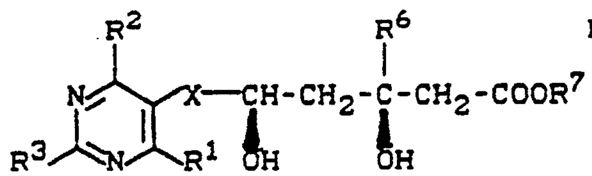

Erythro-Form (IV)

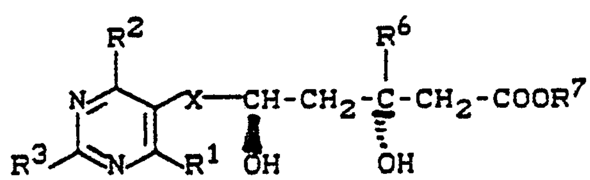

Threo-Form (V)

Sowohl von den Verbindungen in Erythro- als auch in Threo-Konfiguration existieren wiederum jeweils zwei Enantiomere, nämlich 3R,5S-Isomeres bzw. 3S,5R-Isomeres (Erythro-Form) sowie 3R,5R-Isomeres und 3S,5S-Isomeres (Threo-Form).

Bevorzugt sind hierbei die Erythro-konfigurierten Isomeren, besonders bevorzugt das 3R,5S-Isomere sowie das 3R,5S-3S,5R-Racemat.

c) Steht der Rest -A- für eine Gruppe der Formel

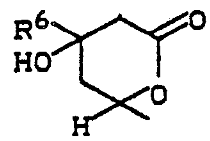

so besitzen die substituierten Pyrimidine mindestens zwei asymmetrische Kohlenstoffatome, nämlich das Kohlenstoffatom an dem die Hydroxygruppe gebunden ist, und das Kohlenstoffatom an welchem der Rest der Formel

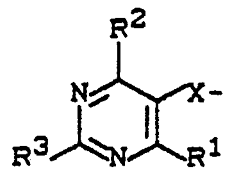

gebunden ist. Je nach der Stellung der Hydroxygruppe zur freien Valenz am Lactonring können die substituierten Pyrimidine als cis-Lactone (VI) oder als trans-Lactone (VII) vorliegen.

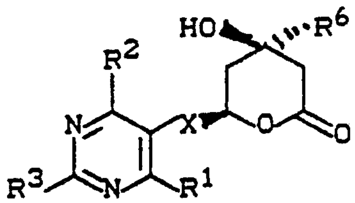

cis-Lacton (VI)

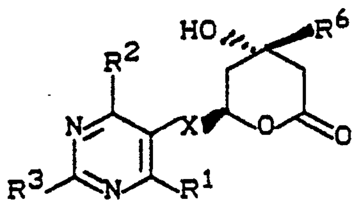

trans-Lacton (VII)

Sowohl vom cis-Lacton aus als auch vom trans-Lacton existieren wiederum jeweils zwei isomeren nämlich das 4R,6R-Isomere bzw. das 4S,6S-Isomere (cis-Lacton), sowie das 4R,6S-Isomere bzw. 4S,6R-Isomere (trans-Lacton). Bevorzugte Isomere sind die trans-Lactone. Besonders bevorzugt ist hierbei das 4R,6S-Isomere (trans) sowie das 4R,6S-4S,6R-Racemat.

Beispielsweise seien die folgenden isomeren Formen der substituierten Pyrimidine genannt:

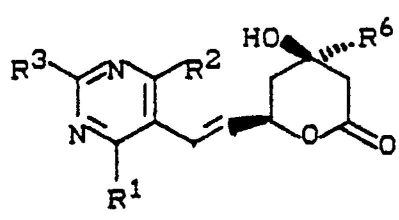

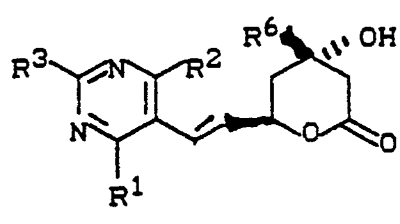

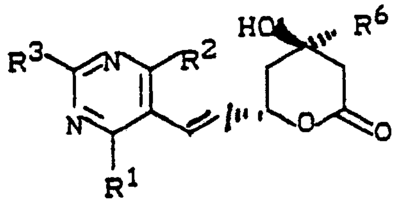

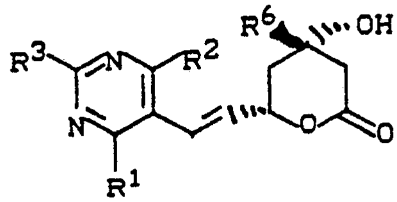

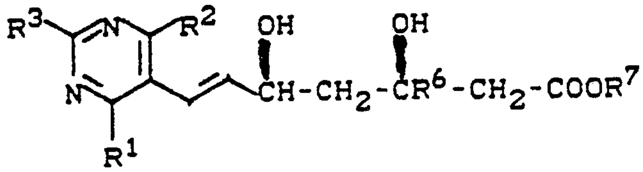

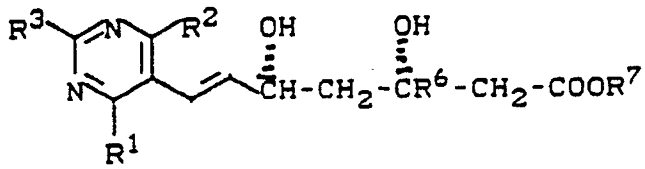

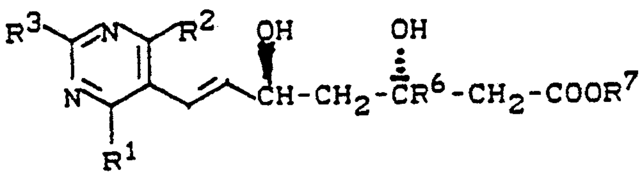

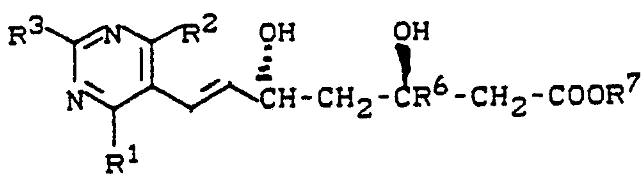

Ganz besonders bevorzugt sind die Verbindungen der allgemeinen Formel I, in welchen

$R^1$ - für Cyclopropyl, Methyl, Ethyl, Propyl, Isopropyl, Butyl, Isobutyl oder tert.Butyl steht,

$R^2$ - für Phenyl steht, das bis zu 2-fach gleich oder verschieden durch Methyl, Methoxy, Phenoxy, Fluor, Chlor oder Trifluormethyl substituiert sein kann,

$R^3$ - für Methyl, Isopropyl, tert.-Butyl oder

- für Phenyl steht, das bis zu 2-fach gleich oder verschieden durch Methyl, Methoxy, Fluor oder Chlor, substituiert sein kann,

X - für eine Gruppe der Formel

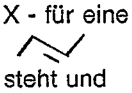 (E-konfiguriert)

steht und

A - für eine Gruppe der Formel

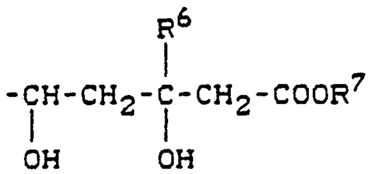 oder 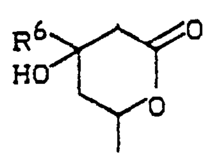

steht, worin
$R^6$ - Wasserstoff bedeutet und
$R^7$ - Wasserstoff, Methyl oder Ethyl bedeutet oder ein Natrium- oder Kaliumkation bedeutet.

Außerdem wurde ein Verfahren zur Herstellung der substituierten Pyrimidine der allgemeinen Formel (I)

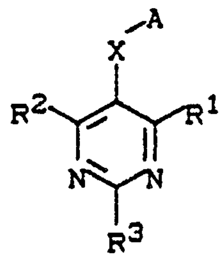

(I)

in welcher
$R^1$, $R^2$, $R^3$, X und A die oben genannte Bedeutung haben,
gefunden, das dadurch gekennzeichnet ist, daß man Ketone der allgemeinen Formel (VIII)

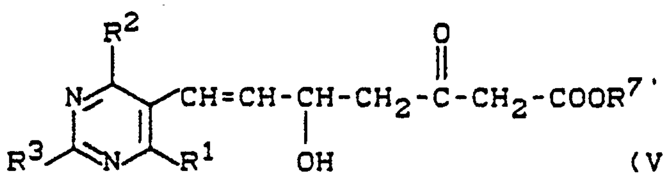

(VIII)

in welcher
$R^1$, $R^2$ und $R^3$ die oben angegebene Bedeutung haben, und
$R^{7'}$ - für Alkyl, Aryl oder Aralkyl steht,
reduziert,
im Fall der Herstellung der Säuren die Ester verseift,
im Fall der Herstellung der Lactone die Carbonsäuren cyclisiert,
im Fall der Herstellung der Salze entweder die Ester oder die Lactone verseift,
im Fall der Herstellung der Ethylenverbindungen (X = $-CH_2-CH_2-$) die Ethenverbindungen (X = -CH=CH-) nach üblichen Methoden hydriert,
und gegebenenfalls Isomeren trennt.

Das erfindungsgemäße Verfahren kann durch das folgendes Formelschema erläutert werden:

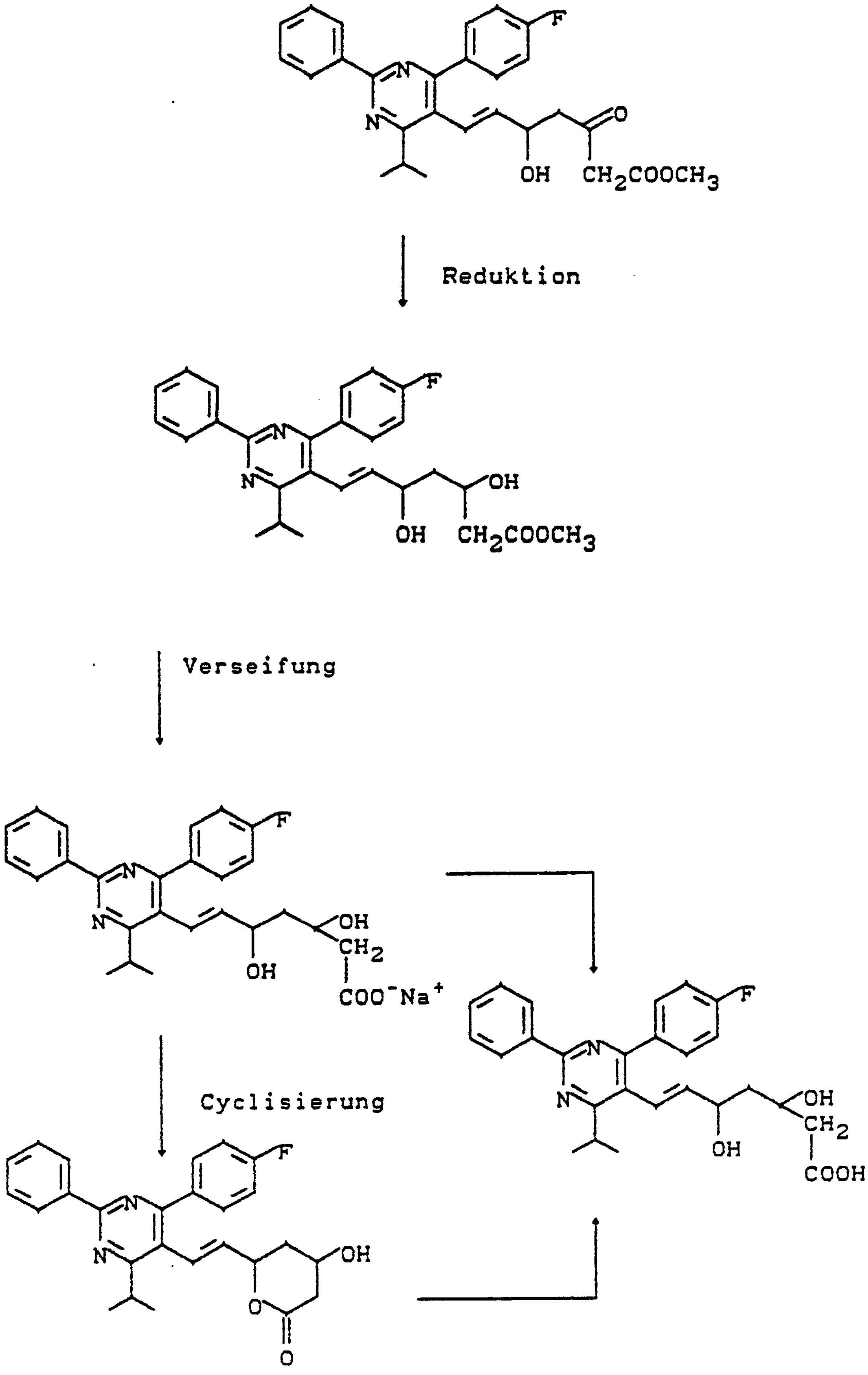


Die Reduktion kann mit den üblichen Reduktionsmitteln, bevorzugt mit solchen, die für die Reduktion von Ketonen zu Hydroxyverbindungen geeignet sind, durchgeführt werden. Besonders geeignet ist hierbei die Reduktion mit Metallhydriden oder komplexen Metallhydriden in inerten Lösemitteln, gegebenenfalls in

Anwesenheit eines Trialkylborans. Bevorzugt wird die Reduktion mit komplexen Metallhydriden wie beispielsweise Lithiumboranat, Natriumboranat, Kaliumboranat, Zinkboranat, Lithium-trialkyl hydrido-borate, Natrium-trialkyl-hydrido-boranaten, Natrium-cyano-trihydrido-borat oder Lithiumaluminiumhydrid durchgeführt. Ganz besonders bevorzugt wird die Reduktion mit Natriumborhydrid, in Anwesenheit von Triethylboran durchgeführt.

Als Lösemittel eignen sich hierbei die üblichen organischen Lösemittel, die sich unter den Reaktionsbedingungen nicht verändern. Hierzu gehören bevorzugt Ether wie beispielsweise Diethylether, Dioxan, Tetrahydrofuran oder Dimethoxyethan, oder Halogenkohlenwassertstoffe wie beispielsweise Dichlormethan, Trichlormethan, Tetrachlormethan, 1,2-Dichlorethan, oder Kohlenwasserstoffe wie beispielsweise Benzol, Toluol oder Xylol. Ebenso ist es möglich Gemische der genannten Lösemittel einzusetzen.

Besonders bevorzugt wird die Reduktion der Ketongruppe zur Hydroxygruppe unter Bedingungen durchgeführt, bei denen die übrigen funktionellen Gruppen wie beispielsweise die Alkoxycarbonylgruppe nicht verändert werden. Hierzu besonders geeignet ist die Verwendung von Natriumborhydrid als Reduktionsmittel, in Anwesenheit von Triethylboran in inerten Lösemitteln wie vorzugsweise Ethern.

Die Reduktion erfolgt im allgemeinen in einem Temperaturbereich von -80 °C bis Raumtemperatur (etwa + 30 °C), bevorzugt von - 78 °C bis 0 °C.

Das erfindungsgemäße Verfahren wird im allgemeinen bei Normaldruck durchgeführt. Es ist aber auch möglich, das Verfahren bei Unterdruck oder bei Überdruck durchzuführen (z.B. in einem Bereich von 0,5 bis 5 bar).

Im allgemeinen wird das Reduktionsmittel in einer Menge von 1 bis 2 mol, bevorzugt von 1 bis 1,5 mol bezogen auf 1 mol der Ketoverbindung eingesetzt.

Unter den ober angegebenen Reaktionsbedingungen wird im allgemeinen die Carbonylgruppe zur Hydroxygruppe reduziert, ohne daß Reduktion an der Doppelbindung zur Einfachbindung erfolgt.

Zur Herstellung von Verbindungen der allgemeinen Formel (I), in welchen X für eine Ethylengruppierung steht, kann die Reduktion der Ketone (III) unter solchen Bedingungen durchgeführt werden, unter denen sowohl die Carbonylgruppe als auch die Doppelbindung reduziert wird.

Darüberhinaus ist es auch möglich, die Reduktion der Carbonylgruppe und die Reduktion der Doppelbindung in zwei getrennten Schritten durchzuführen.

Die Carbonsäuren im Rahmen der allgemeinen Formel (I) entsprechen der Formel (Ic)

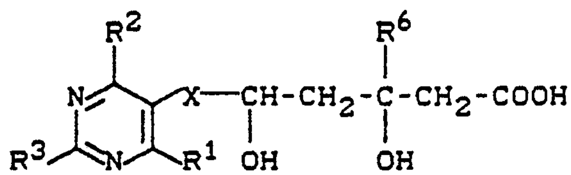

(Ic)

Die Carbonsäureester im Rahmen der allgemeinen Formel (I) entsprechen der Formel (Id)

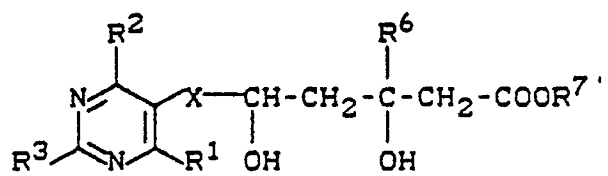

(Id)

Die Salze der erfindungsgemäßen Verbindungen im Rahmen der allgemeinen Formel (I) entsprechen der Formel (Ie)

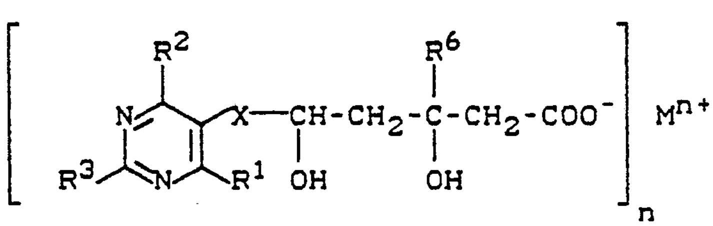

(Ie)

in welcher
$M^{n+}$ für ein Kation mit der Wertigkeit n steht. Die bevorzugte Wertigkeit ist 1 oder 2.

Die Lactone im Rahmen der allgemeinen Formel (I) entsprechen der Formel (If)

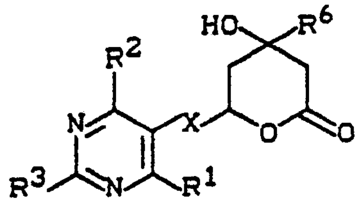

(If)

Zur Herstellung der erfindungsgemäßen Carbonsäuren der allgemeinen Formel (Ic) werden im allgemeinen die Carbonsäureester der allgemeinen Formel (Id) oder die Lactone der allgemeinen Formel (If) nach üblichen Methoden verseift. Die Verseifung erfolgt im allgemeinen indem man die Ester oder die Lactone in inerten Lösemitteln mit üblichen Basen behandelt, wobei im allgemeinen zunächst die Salze der allgemeinen Formel (Ie) entstehen, die anschließend in einem zweiten Schritt durch Behandeln mit Säuren in die freien Säuren der allgemeinen Formel (Ic) überführt werden können.

Als Basen eignen sich für die Verseifung die üblichen anorganischen Basen. Hierzu gehören bevorzugt Alkalihydroxide oder Erdalkalihydroxide wie beispielsweise Natriumhydroxid, Kaliumhydroxid oder Bariumhydroxid, oder Alkalicarbonate wie Natrium - oder Kaliumcarbonat oder Natriumhydrogencarbonat, oder Alkalialkoholate wie Natriumethanolat, Natriummethanolat, Kaliummethanolat, Kaliumethanolat oder Kalium-tert.butanolat. Besonders bevorzugt werden Natriumhydroxid oder Kaliumhydroxid eingesetzt.

Als Lösemittel eigen sich für die Verseifung Wasser oder die für eine Verseifung üblichen organischen Lösemittel. Hierzu gehören bevorzugt Alkohole wie Methanol, Ethanol, Propanol, Isopropanol oder Butanol, oder Ether wie Tetrahydrofuran oder Dioxan, oder Dimethylformamid oder Dimethylsulfoxid. Besonders bevorzugt werden Alkohole wie Methanol, Ethanol, Propanol oder Isopropanol verwendet. Ebenso ist es möglich, Gemische der genannten Lösemittel einzusetzen.

Die Verseifung wird im allgemeinen in einem Temperaturbereich von 0° C bis +100° C, bevorzugt von + 20° C bis +80° C durchgeführt.

Im allgemeinen wird die Verseifung bei Normaldruck durchgeführt. Es ist aber auch möglich, bei Unterdruck oder bei Überdruck zu arbeiten (z.B. von 0,5 bis 5 bar).

Bei der Durchführung der Verseifung wird die Base im allgemeinen in einer Menge von 1 bis 3 mol, bevorzugt von 1 bis 1,5 mol bezogen auf 1 mol des Esters bsw. des Lactons eingesetzt. Besonders bevorzugt verwendet man molare Mengen der Reaktanden.

Bei der Durchführung der Reaktion entstehen im ersten Schritt die Salze der erfindungsgemäßen Verbindungen (Ie) als Zwischenprodukte, die isoliert werden können. Die erfindungsgemäßen Säuren (Ic) erhält man durch Behandeln der Salze (Ie) mit üblichen anorganischen Säu ren. Hierzu gehören bevorzugt Mineralsäuren wie beispielsweise Chlorwasserstoffsäure, Bromwasserstoffsäure, Schwefelsäure oder Phosphorsäure. Es hat sich bei der Herstellung der Carbonsäuren (Ic) hierbei als vorteilhaft erwiesen, die basische Reaktionsmischung der Verseifung in einem zweiten Schritt ohne Isolierung der Salze anzusäuern. Die Säuren können dann in üblicher Weise isoliert werden.

Zur Herstellung der erfindungsgemäßen Lactone der Formel (If) werden im allgemeinen die erfindungsgemäßen Carbonsäuren (Ic) nach üblichen Methoden cyclisiert, beispielsweise durch Erhitzen der entsprechenden Säure in inerten organischen Lösemitteln, gegebenenfalls in Anwesenheit von Molsieb.

Als Lösemittel eignen sich hierbei Kohlenwasserstoffe wie Benzol, Toluol, Xylol, Erdölfraktionen, oder Tetralin oder Diglyme oder Triglyme. Bevorzugt werden Benzol, Toluol oder Xylol eingesetzt. Ebenso ist es möglich Gemische der genannten Lösemittel einzusetzen. Besonders bevorzugt verwendet man Kohlenwasserstoffe, insbesondere Toluol, in Anwesenheit von Molsieb.

Die Cyclisierung wird im allgemeinen in einem Temperaturbereich von -40° C bis +200° C, bevorzugt von -25° C bis +50° C durchgeführt.

Die Cyclisierung wird im allgemeinen bei Normaldruck durchgeführt, es ist aber auch möglich, das Verfahren bei Unterdruck oder bei Überdruck durchzuführen (z.B. in einem Bereich von 0,5 bis 5 bar).

Darüberhinaus wird die Cyclisierung auch in inerten organischen Lösemitteln, mit Hilfe von cyclisierenden bzw. wasserabspaltenden Agentien durchgeführt. Als wasserabspaltende Agentien werden hierbei bevorzugt Carbodiimide verwendet. Als Carbodiimide werden bevorzugt N,N′-Di-cyclohexylcarbodiimid-Paratoluolsulfonat, N-Cyclohexyl-N′-[2-(N″-methylmorpholinium)ethyl]carbodiimid oder N-(3-Dimethylaminopropyl)-N′- ethylcarbodiimid-Hydrochlorid eingesetzt.

Als Lösemittel eignen sich hierbei die üblichen organischen Lösemittel. Hierzu gehören bevorzugt Ether wie Diethylether, Tetrahydrofuran oder Dioxan, oder Chlorkohlenwasserstoffe wie Methylenchlorid, Chloroform oder Tetrachlorkohlenstoff, oder Kohlenwasserstoffe wie Benzol, Toluol, Xylol oder Erdölfraktionen. Besonders bevorzugt werden Chlorkohlenwasserstoffe wie beispielsweise Methylenchlorid, Chloroform oder Tetrachlorkohlenstoff, oder Kohlenwasserstoffe wie Benzol, Toluol, Xylol, oder Erdölfraktionen. Besonders bevorzugt werden Chlorkohlenwasserstoffe wie beispielsweise Methylenchlorid, Chloroform oder Tetrachlorkohlenstoff eingesetzt.

Die Reaktion wird im allgemeinen in einem Temperaturbereich von 0°C bis +80°C, bevorzugt von +10°C bis +50°C durchgeführt.

Bei der Durchführung der Cyclisierung hat es sich als vorteilhaft erwiesen, die Cyclisierungsmethode mit Hilfe von Carbodiimiden als dehydratisierenden Agentien einzusetzen.

Die Trennung der Isomeren in die stereoisomer einheitlichen Bestandteile erfolgt im allgemeinen nach üblichen Methoden wie sie beispielsweise von E.L. Eliel, Stereochemistry of Carbon Compounds, McGraw Hill, 1962 beschrieben wird, Bevorzugt wird hierbei die Trennung der Isomeren auf der Stufe der racemischen Lactone. Besonders bevorzugt wird hierbei die racemische Mischung der trans-Lactone (VII) durch Behandeln entweder mit (D-(+)-oder L-(-)-α-Methylbenzylamin nach üblichen Methoden in die diastereomeren Dihydroxyamide (Ig)

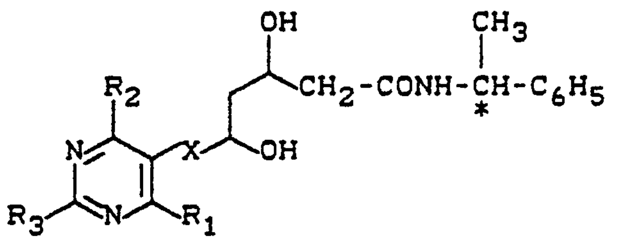

(Ig)

überführt, die anschließend wie üblich durch Chromatographie oder Kristallisation in die einzelnen Diastereomeren getrennt werden können. Anschließende Hydrolyse der reinen diastereomeren Amide nach üblichen Methoden, beispielsweise durch Behandeln der diastereomeren Amide mit anorganischen Basen wie Natriumhydroxid oder Kalium hydroxid in Wasser und/oder organischen Lösemitteln wie Alkoholen z.B. Methanol, Ethanol, Propanol oder Isopropanol, ergeben die entsprechenden enantiomerenreinen Dihydroxysäuren (Ic), die wie oben beschrieben durch Cyclisierung in die enantiomerenreinen Lactone überführt werden können. Im allgemeinen gilt für die Herstellung der erfindungsgemäßen Verbindungen der allgemeinen Formel (I) in enantiomerenreiner Form, daß die Konfiguration der Endprodukt nach der oben beschriebenen Methode abhängig ist von der Konfiguration der Ausgangsstoffe.

Die Isomerentrennung soll im folgenden Schema beispielhaft erläutert werden:

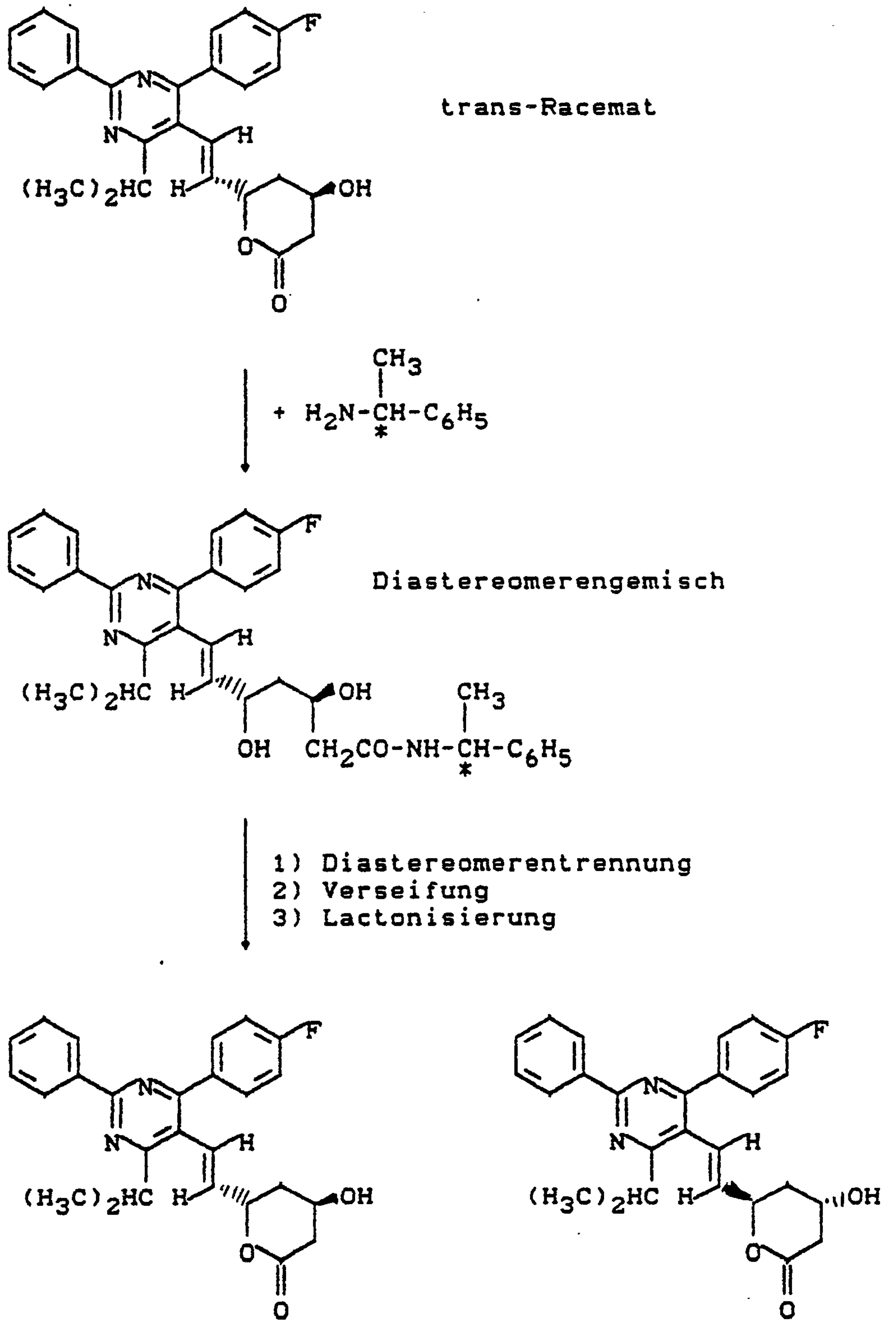


Die als Ausgangsstoffe eingesetzten Ketone (VIII) sind neu.

Es wurde ein Verfahren zur Herstellung der erfindungsgemäßen Ketone der allgemeinen Formel (VIII)

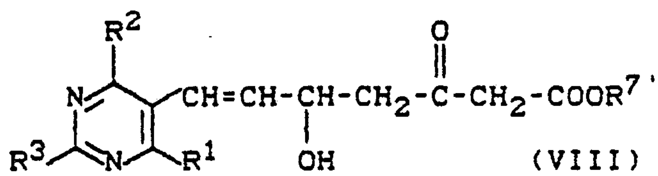

(VIII)

in welcher
$R^1$, $R^2$, $R^3$ und $R^{7'}$ die oben genannte Bedeutung haben,
gefunden, das dadurch gekennzeichnet ist, daß man Aldehyde der allgemeinen formel (IX)

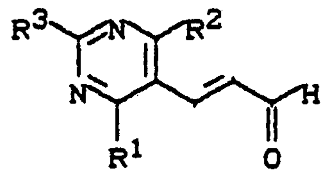

(IX)

in welcher
$R^1$, $R^2$ und $R^3$ die oben angegebene Bedeutung haben,
in inerten Lösemitteln mit Acetessigester der allgemeinen formel (X)

$H_3C-\overset{O}{\overset{\|}{C}}-CH_2-COOR^{7'}$ (X)

in welcher
$R^{7'}$ die oben angegebene Bedeutung hat,
in Anwesenheit von Basen umsetzt.

Das erfindungsgemäße Verfahren kann beispielsweise durch folgendes Formelschema erläutert werden:

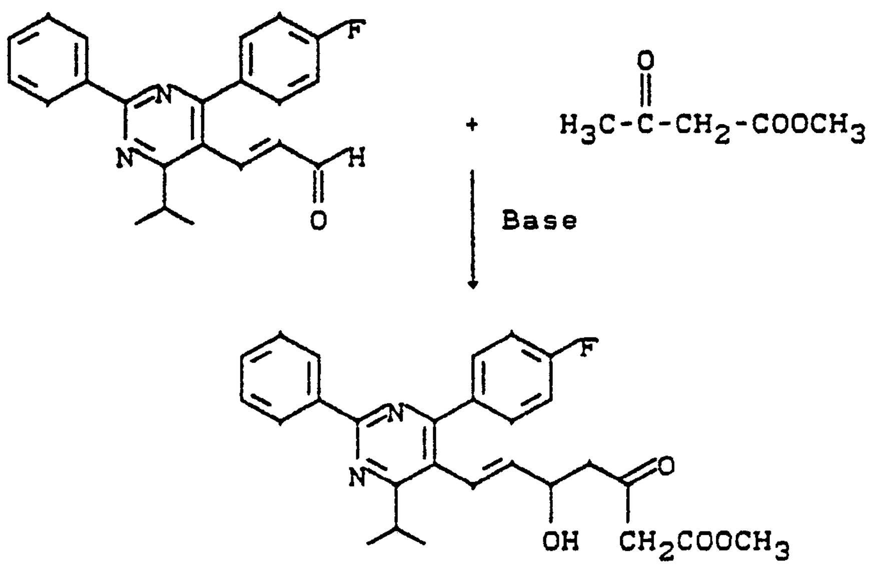


Als Basen kommen hierbei die üblichen stark basischen Verbindungen in Frage. Hierzu gehören bevorzugt lithiumorganische Verbindungen wie beispielsweise N-Butyllithium, sec.Butyllithium, tert.Butyllithium oder Phenyllithium, oder Amide wie beispielsweise Lithiumdiisopropyl amid, Natriumamid oder Kaliumamid, oder Lithiumhexamethyldisilylamid, oder Alkalihydride wie Natriumhydrid oder Kaliumhydrid. Ebenso ist es möglich, Gemische der genannten Basen einzusetzen. Besonders bevorzugt werden N-Butyllithium oder Natriumhydrid oder deren Gemisch eingesetzt.

Als Lösemittel eignen sich hierbei die üblichen organischen Lösemittel, die sich unter den Reaktionsbedingungen nicht verändern. Hierzu gehören bevorzugt Ether wie Diethylether, Tetrahydrofuran, Dioxan oder Dimethoxyethan, oder Kohlenwasserstoffe wie Benzol, Toluol, Xylol, Cyclohexan, Hexan oder Erölfraktionen. Ebenso ist es möglich, Gemische der genannten Lösemittel einzusetzen. Besonders bevorzugt werden Ether wie Diethylether oder Tetrahydrofuran verwendet.

Die Reaktion wird im allgemeinen in einem Temperaturbereich von -80 °C bis +50 °C, bevorzugt von -20 °C bis Raumtemperatur durchgeführt.

Das Verfahren wird im allgemeinen bei Normaldruck durchgeführt, es ist aber auch möglich das Verfahren bei Unterdruck oder bei Überdruck durchzuführen, z.B. in einem Bereich von 0,5 bis 5 bar.

Bei der Durchführung des Verfahrens wird der Acetessigester im allgemeinen in einer Menge von 1 bis 2, bevorzugt von 1 bis 1,5 mol, bezogen auf 1 mol des Aldehyds eingesetzt.

Die als Ausgangsstoffe eingesetzten Acetessigester der Formel (X) sind bekannt oder können nach bekannten Methoden hergestellt werden [Beilstein's Handbuch der organischen Chemie III, 632; 438].

Als Acetessigester für das erfindungsgemäße Verfahren seien beispielsweise genannt:

Acetessigsäuremethylester, Acetessigsäureethylester, Acetessigsäurepropylester, Acetessigsäureisopropylester.

Die als Ausgangsstoffe eingesetzten Aldehyde der allgemeinen formel (IX) sind neu.

Es wurde außerdem ein Verfahren zur Herstellung der Aldehyde der allgemeinen Formel (IX)

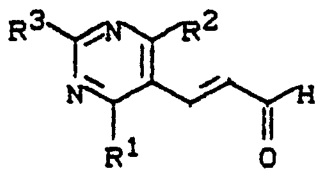

(IX)

in welcher

$R^1$, $R^2$, $R^3$ die oben angegebene Bedeutung haben,

gefunden, das dadurch gekennzeichnet ist, daß man Pyrimidine der allgemeinen formel (XI) im Temperaturbereich von -70 °C bis +100 °C zu Hydroxymethylverbindungen der Formel (XII) reduziert, anschließend zu den entsprechenden Aldehyden (XIII) oxidiert und diese mit Dialkyl-($C_1$ bis $C_4$)-aminovinylphosphonaten in Anwesenheit von Natriumhydrid in inerten Lösungsmitteln wie Ethern, beispielsweise Diethylether, Tetrahydrofuran oder Dioxan, vorzugsweise Tetrahydrofuran in einem Temperaturbereich von -20 °C bis +40 °C, vorzugsweise von -5 °C bis Raumtemperatur zu den Aldehyden (IX) umsetzt.

Ihre Herstellung soll im folgenden beispielhaft für die Verbindungen des Typs (I) erläutert werden.

(A)

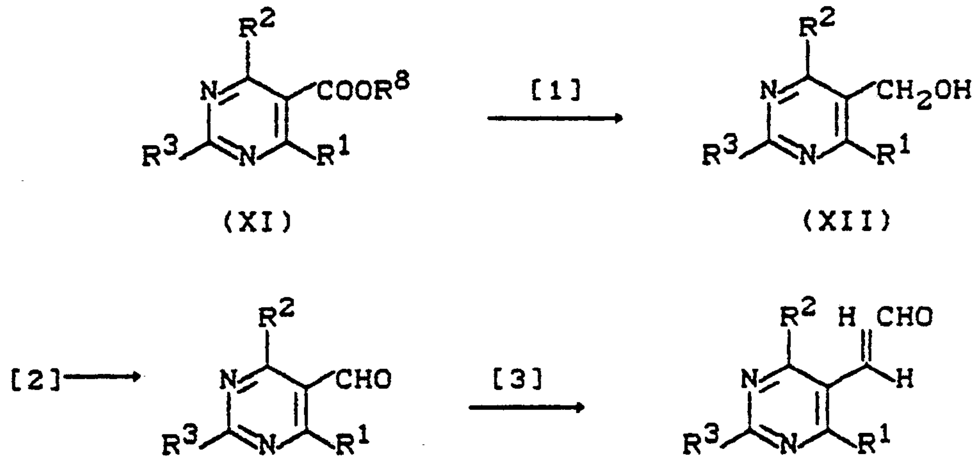


(XI) (XII)

(XIII) (IX)

Hierbei werden gemäß Schema A Pyrimidine der Formel (XI), in welchen

$R^8$ für einen Alkyl mit bis zu 6 Kohlenstoffatomen, Aryl ($C_6$ bis $C_{12}$) oder Aralkyl ($C_7$ bis $C_{18}$) steht, und $R^1$, $R^2$ und $R^3$ die oben genannte Bedeutung haben, im ersten Schritt (1) in inerten Lösemitteln wie Ethern, beispielsweise Diethylether, Tetrahydrofuran oder Dioxan, vorzugsweise Tetrahydrofuran oder Dioxan, vorzugsweise Tetrahydrofuran, mit Metallhydriden als Reduktionsmittel, beispielsweise Lithiumaluminiumhydrid, Natriumcyanoborhydrid, Natriumaluminiumhydrid, Diisobutylaluminiumhydrid oder Natrium-bis-(2-methoxyethoxy)-dihydroaluminat, in Temperaturbereichen von -70 °C bis +100 °C, vorzugsweise von -70 °C bis Raumtemperatur, bsw. von Raumtemperatur bis 70 °C je nach verwendetem Reduktionsmittel zu den Hydroxymethylverbindungen (XII) reduziert. Vorzugsweise wird die Reduktion mit Lithiumaluminiumhydrid in Tetrahydrofuran in einem Temperaturbereich von Raumtemperatur bis 80 °C durchgeführt. Die Hydroxymethylverbindungen (XII) werden im zweiten Schritt (2) nach üblichen Methoden zu den Aldehyden (XII) oxidiert. Die Oxidation kann beispielsweise mit Pyridiniumchlorochromat, gegebenenfalls in Anwesenheit von Aluminiumoxid, in inerten Lösemitteln wie Chlorkohlenwasserstoffen, vorzugsweise Methylenchlorid, in

einem Temperaturbereich von 0°C bis 60°C, bevorzugt bei Raumtemperatur durchgeführt werden, oder aber mit Trifluoressigsäure/Dimethylsulfoxid nach den üblichen Methoden der Swern Oxidation durchgeführt werden. Die Aldehyde (XIII) werden im dritten Schritt (3) bevorzugt mit Diethyl-2-(cyclohexylamino)-vinylphosphonat in Anwesenheit von Natriumhydrid in inerten Lösemitteln wie Ethern, beispielsweise Diethylether, Tetrahydrofuran oder Dioxan, vorzugsweise in Tetrahydrofuran, in einem Temperaturbereich von -20°C bis +40°C, vorzugsweise von -5°C bis Raumtemperatur zu den Aldehyden (IX) umgesetzt.

Die hierbei als Ausgangsstoffe eingesetzten Pyrimidine der Formel (XI) erhält man hierbei im allgemeinen gemäß Schema B durch Oxidation von Dihydropyrimidinen (XIV). Die hierbei als Ausgangsstoffe eingesetzten Dihydropyrimidine werden nach literaturbekannten Methoden hergestellt (DE-A 103 796). Die Oxidation der Dihydropyrimidine (XIV) zu den Pyrimidinen (XI kann beispielsweise mit Chromoxid in Eisessig in einem Temperaturbereich von -20°C bis +150°C, vorzugsweise bei Rückflußtemperatur, oder mit 2,3-Dichlor-5,6-dicyan-p-benzochinon als Oxidationsmittel in inerten Lösungsmitteln wie Chlorkohlenwasserstoffe, vorzugsweise Methylenchlorid in einem Temperaturbereich von 0°C bis +100°C, vorzugsweise bei Raumtemperatur durchgeführt werden.

(B)

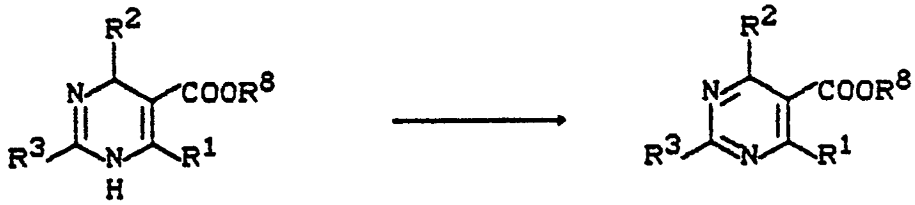

(XIV) (XI)

Die erfindungsgemäßen Verbindungen der allgemeinen Formel (I) besitzen wertvolle pharmakologische Eigenschaften und können als Wirkstoffe in Arzneimitteln eingesetzt werden. Insbesondere sind sie Inhibitoren der 3-Hydroxy-3-methyl-glutaryl-Coenzym A (HMG-CoA) Reduktase und infolge dessen Inhibitoren der Cholesterolbiosynthese. Sie können deshalb zur Behandlung von Hyperlipoproteinämie, Lipoproteinämie oder Arteriosklerose eingesetzt werden.

Die neuen Wirkstoffe können in bekannter Weise in die üblichen Formulierungen überführt werden, wie Tabletten, Dragees, Pillen, Granulate, Aerosole, Sirupe, Emulsionen, Suspensionen und Lösungen, unter Verwendung inerter, nicht toxischer, pharmazeutisch geeigneter Trägerstoffe oder Lösungsmittel. Hierbei soll die therapeutisch wirksame Verbindung jeweils in einer Konzentration von etwa 0,5 bis 98-Gew.-%, bevorzugt 1 bis 90 Gew.-%, der Gesamtmischung vorhanden sein, d.h. in Mengen, die ausreichend sind, um den angegebenen Dosierungsspielraum zu erreichen.

Die Formulierungen werden beispielsweise hergestellt durch Verstrecken der Wirkstoffe mit Lösungsmitteln und/oder Trägerstoffen, gegebenenfalls unter Verwendung von Emulgiermitteln und/oder Dispergiermitteln, wobei z.B. im Fall der Benutzung von Wasser als Verdünnungsmittel gegebenenfalls organische Lösungsmittel als Hilfslösungsmittel verwendet werden können.

Als Hilfsstoffe seien beispielsweise aufgeführt: Wasser, nicht-toxische organische Lösungsmittel, wie

Paraffine (z.B. Erdölfraktionen), pflanzliche Öle (z.B. Erdnuß/Sesamöl), Alkohole (z.b: Ethylalkohol, Glycerin), Trägerstoffe, wie z.B. natürliche Gesteinsmehle (z.B. Kaoline, Tonerden, Talkum, Kreide), synthetische Gesteinsmehle (z.B. hochdisperse Kieselsäure, Silikate), Zucker (z.B. Rohr-, Milch- und Traubenzukker), Emulgiermittel (z.B. Polyoxyethylen-Fettsäure-Ester, Polyoxyethylen-Fettalkohol-Ether, Alkylsulfonate und Arylsulfonate), Dispergiermittel (z.B. Lignin-Sulfitablaugen, Methylcellulose, Stärke und Polyvinylpyrrolidon) und Gleitmittel (z.B. Magnesiumstearat, Talkum, Stearinsäure und Natriumlaurylsulfat).

Die Applikation erfolgt in üblicher Weise, vorzugsweise oral, parenteral, perlingual oder intravenös. Im Falle der oralen Anwendung können Tabletten selbstverständlich außer den genannten Trägerstoffen auch Zusätze, wie Natriumcitrat, Calciumcarbonat und Dicalciumphosphat zusammen mit verschiedenen Zuschlagstoffen, wie Stärke, vorzugsweise Kartoffelstärke, Gelatine und dergleichen enthalten. Weiterhin können Gleitmittel, wie Magnesiumstearat, Natriumlaurylsulfat und Talkum zum Tablettieren mitverwendet werden. Im Falle wäßriger Suspensionen können die Wirkstoffe außer den obengenannten Hilfsstoffen mit verschiedenen Geschmacksaufbesserern oder Farbstoffen versetzt werden.

Für den Fall der parenteralen Anwendung können Lösungen der Wirkstoffe unter Verwendung geeigneter flüssiger Trägermaterialien eingesetzt werden.

Im allgemeinen hat es sich als vorteilhaft erwiesen, bei intravenöser Applikation Mengen von etwa 0,001 bis 1 mg/kg, vorzugsweise etwa 0,01 bis 0,5 mg/kg Körpergewich zur Erzielung wirksamer Ergebnisse zu verabreichen, und bei oraler Applikation beträgt die Dosierung etwa 0,01 bis 20 mg/kg, vorzugsweise 0,1 bis 10 mg/kg Körpergewicht.

Trotzdem kann es gegebenenfalls erforderlich sein, von den genannten Mengen abzuweichen, und zwar in Abhängigkeit vom Körpergewicht bzw. der Art des Applikationsweges, vom individuellen Verhalten gegenüber dem Medikament, der Art von dessen Formulierung und dem Zeitpunkt bzw. Intervall, zu welchen die Verabreichung erfolgt. So kann es in einigen Fällen ausreichend sein, mit weniger als der vorgenannten Mindestmenge auszukommen, während in anderen Fällen die genannte obere Grenze überschritten werden muß. Im Falle der Applikation größerer Mengen kann es empfehlenswert sein, diese in mehreren Einzelgaben über den Tag zu verteilen.

Beispiel 1

(E/Z)-3-Ethoxycarbonyl-4-(4-fluorphenyl)-but-3-en-2-on

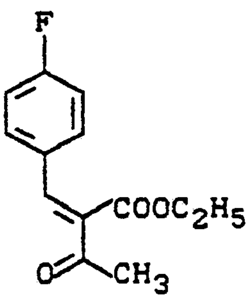

62 g (0,5 mol) 4-Fluorbenzaldehyd und 53,9 ml (0,5 mol) Acetessigsäureethylester werden in 300 ml Isopropanol vorgelegt, mit einem Gemisch aus 2,81 ml (28 mmol) Piperidin und 1,66 ml (19 mmol) Essigsäure in 40 ml Isopropanol versetzt und 48 h bei Raumtemperatur gerührt. Die Mischung wird im Vakuum eingeengt und der Rückstand im Hochvakuum destilliert.
Kp 0,5 mm: 138 °C
Ausbeute: 50,5 g (45,5 % der Theorie).

Beispiel 2

1,4-Dihydro-2,6-dimethyl-4-(4-fluorphenyl)-pyrimidin-5-carbonsäureethylester

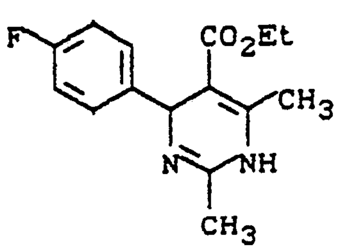

In 230 ml Ethanol werden 23,6 g (0,1 mol) Beispiel 1, 10,5 g (0,11 mol) Acetamidinhydrochlorid und 9,85 g (0,12 mol) Natriumacetat über Nacht unter Rückfluß erhitzt, das Lösungsmittel im Vakuum entfernt, der Rückstand mit Essigsäureethylester und 180 ml 1N-Salzsäure versetzt, die organische Phase noch zweimal mit 100 ml 1N-Salzsäure gewaschen, die vereinigten Wäßrigen Phasen mit Ether gewaschen, mit konzentrierter Natronlauge alkalisch gestellt und dreimal mit Essigester extrahiert. Die Essigesterphase wird

mit Wasser gewschen, getrocknet ($Na_2SO_4$) und im Vakuum eingeengt. Ausbeute: 12,4 g 44,9 % der Theorie
$^1$H-NMR ($CDCl_3$) : δ = 1,15(tr, 3H, $CH_3$); 1,9 (s, 3H, $CH_3$); 2,3 (s, 3H, $CH_3$), 4,05 (m, 2H, $CH_2$); 5,3, 5,5 (2s, 1H, CH); 6,9-7,4 (m, 4H, Aromaten-H) ppm.

Beispiel 3

2,6-Dimethyl-4-(4-fluorphenyl)-pyrimidin-5-carbonsäureethylester

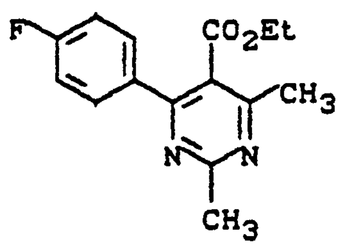

In 70 ml Essigsäure werden 12,4 g (45,1 mol) Beispiel 2, zum Sieden erhitzt und 4,6 g (46 mmol) Chromtrioxid portionsweise hinzugegeben. Nach einer Stunde läßt man auf 25 °C abkühlen, gießt auf Eis/25 % wäßrige Ammoniaklösung, stellt mit Ammoniaklösung alkalisch, extrahiert mit Dichlormethan, wäscht die organische Phase mit Wasser und erhält nach Trocknen ($Na_2SO_4$), Einengen im Vakuum und Filtration über Kieselgel (Dichlormethan/Methanol 10:1) 7,14 g.
Ausbeute: 57,7 % der Theorie
$^1$H-NMR ($CDCl_3$): δ = 1,2 (tr, 3H, $CH_3$); 2,65 (s, 3H, $CH_3$); 2,85 (s, 3H, $CH_3$); 4,3 (q, 2H, $CH_2$); 7,1-7,8 (m 4H, Aromaten-H) ppm.

Beispiel 4

2,6-Dimethyl-4-(4-fluorphenyl)-5-hydroxymethyl-pyrimidin

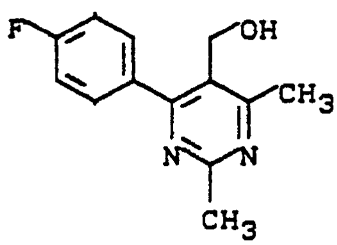

Zu 5,66 g (20,6 mmol) Beispiel 3 in 100 ml Toluol tropft man unter Stickstoffatmosphäre bei -78 °C 27 ml (40 mmol) Diisobutylaluminiumhydrid (1,5 m in Toluol). Nach dem Aufwärmen auf 25 °C wird mit 20 %iger wäßrige Kaliumhydroxidlösung hydrolysiert, die wäßrige Phase mit Essigester gewaschen, die vereinigten organischen Phasen getrocknet ($Na_2SO_4$) und das Lösungsmittel im Vakuum entfernt.
Ausbeute: 4,1 g 85,9 % der Theorie
$^1$H-NMR ($CDCl_3$) : δ = 1,8 (tr, 1H, OH); 2,7 (2s, 6H, $CH_3$); 4,65 (d, 2H, $CH_2$): 7,1-7,7 (m, 4H Aromaten-H)-ppm.

Beispiel 5

2,6-Dimethyl-4(4-fluorphenyl)-pyrimidin-5-carbaldehyd

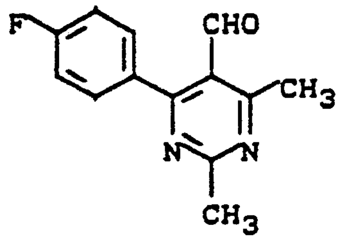

Zu einer Lösung von 4,1 g (17,7 mmol) Beispiel 4 in 100 ml Dichlormethan bei Raumtempertaur gibt man portionweise 6,06 g (28,1 mmol) Pyrimidiniumchlorochromat und rührt zwei Stunden. Das Lösungsmittel wird im Vakuum entfernt und der Rückstand über Kieselgel (Dichlormethan/Methanol 3:1) filtriert.
Ausbeute: 3,8 g 93,4 % der Theorie
$^1$H-NMR ($CDCl_3$) : δ = 2,8 (2s, 6H, $CH_3$); 7,1-7,7 (m, 4H, Aromaten-H); 10,0 (s, 1H, CHO) ppm.

Beispiel 6

(E)-3-[2,6-Dimethyl-4(4-fluorphenyl)-pyrimid-5-yl)]-prop-2-enal

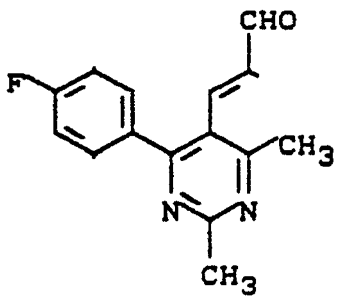

Zu einer Suspension von 0,5 g (20,8 mmol) Natriumhydrid in 20 ml THF tropft man bei 0 °C eine Lösung von 0,81 g [2-(Cyclohexylamino)vinyl]-phosphonsäurediethylester in 20 ml THF, rührt 15 min und tropft dann eine Lösung von 0,63 g (2,72 mmol) Beispiel 5 in 15 ml THF hinzu, rührt 1 h bei 0 °C, 1 h bei 25 °C, hydrolysiert mit 20 ml Wasser, wäscht mit diethylether, entfernt das Lösungsmittel im Vakuum, nimmt den Rückstand in 40 ml Toluol auf und erhitzt die Lösung mit 2 g Oxalsäure und 40 ml Wasser 1 h bei 60 °C, wäscht mit Diethylether, trocknet ($Na_2SO_4$) und erhält nach Entfernen des Lösungsmittels 0,5 g.
Ausbeute: 71,7 % der Theorie
$^1$H-NMR ($CDCl_3$) : δ = 2,7 (s, 3H, $CH_3$); 2,8 (s, 3H, $CH_3$); 6,4 (dd, 1H, CHCHO); 7,2 (d, 1H, CH); 7,1-7,7 (m, 4H, Aromaten-H); 9,6 (d, 1H, CHO) ppm.

Beispiel 7

Methyl(E)-7-[2,6-dimethyl-4-(4-fluorphenyl)-pyrimid-5-yl]-5-hydroxy-3-oxo-hept-6-enoat

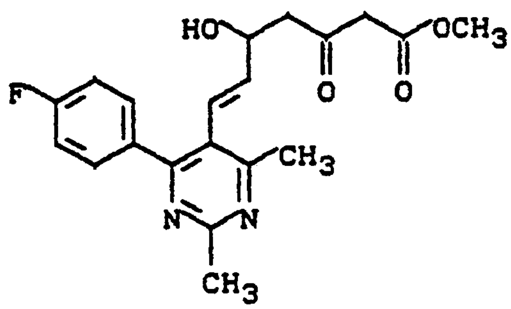

Zu einer Suspension aus 256 mg (10,7 mmol) Natriumhydrid in 25 ml THF tropft man bei 0 °C, 1,11 ml (10,3 mmol) Acetessigsäuremethylester. Nach 15 min tropft man innerhalb 10 min 7,3 ml (10,7 mmol) n-Butyllithium (1,5 m in Hexan) zu, rührt 15 min bei 0 °C, tropft eine Lösung von 2,4 g (3,8 mmol) Beispiel 6 in 10 ml THF zu, rührt 15 min bei 0 °C, hydrolysiert mit 30 ml gesättigter wäßrige Ammoniumchloridlösung, wäscht dreimal mit Dichlormethan, trocknet ($Na_2SO_4$), entfernt das Lösungsmittel im Vakuum und erhält nach Chromatographie über Kieselgel (Essigsäureethylester) 2,47 g Öl.
Ausbeute: 73,5 % der Theorie
$^1$H-NMR ($CDCl_3$) : δ = 2,55 (s, 3H, $CH_3$; 2,7 (d, 2H, <u>$CH_2$</u>-CHOH); 2,75 (s, 3H, $CH_3$); 3,1 (br, s, 1H, OH); 3,5 (s, 2H, $CH_2$); 3,75 (s, 3H, $OCH_3$); 4,65 (m, 1H, CHOH); 5,6 (dd, 1H, <u>CH</u>CHOH); 6,6 (d, 1H, CH); 7,0-7,6 (m, 4H, Aromaten-H) ppm.

<u>Beispiel 8</u>

Methyl-erythro-(E)-3,5-dihydroxy-7-[2,6-dimethyl-4-(4-fluorphenyl)-pyrimid-5-yl]-hept-6-enoat

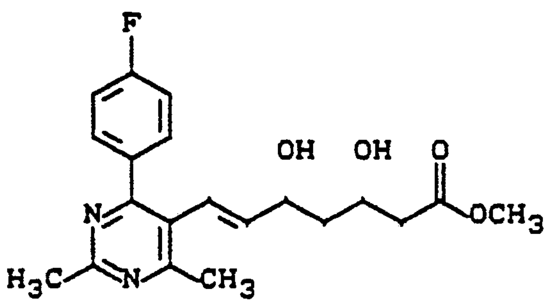

Durch eine Lösung von 1,69 g (4,55 mmol) Beispiel 7 und 5,7 ml (5,7 mmol) Triethylboran (1 m, THF) in 45 ml THF bläst man 5 min Luft, kühlt auf -30 °C, gibt 215 mg (5,7 mmol) Natriumborhydrid und langsam 3,8 ml Methanol hinzu, rührt 30 min bei -30 °C, gibt langsam eine Lösung von 15,2 ml Wasserstoffperoxid (30 %ig) in 33,4 ml Wasser unterhalb 0 °C hinzu, rührt 30 min bei 0 °C, nimmt in Essigsäureester auf, wäscht mit Wasser, 1N Salzsäure, wäßrige Natriumhydrogencarbonatlösung, trocknet ($Na_2SO_4$), entfernt das Lösungsmittel im Vakuum und erhält nach Chromatographie über Kieselgel (Essigsäureethylester) 1,08 g.
Ausbeute: 63 % der Theorie
$^1$H-NMR ($CDCl_3$) : δ = 1,8 (br, s, 2H, OH); 2,5 (m, 2H, $CH_2$); 2,55 (s, 3H, $CH_3$); 2,7 (s, 3H, $CH_3$); 3,7 (s, 3H, $OCH_3$); 4,2 (m, 1H, CHOH); 4,45 (m, 1H, CHOH); 5,6 (dd, 1H, <u>CH</u>, CHOH); 6,5 (d, 1H, CH); 7,0-7,6 (m, 4H, Aromaten-H) ppm.

<u>Beispiel 9</u>

1,4-Dihydro-4-(4-fluorphenyl)-6-methyl-2-phenyl-pyrimidin-5-carbonsäureethylester

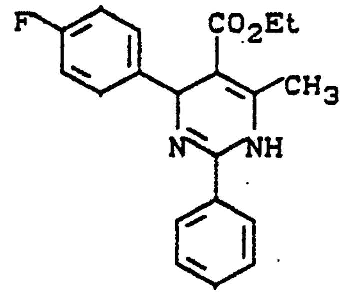

Analog Beispiel 2 erhält man aus 103,6 g (0,56 mol) Benzamidinhydrochlorid, 121,5 g (0,5 mol) Beispiel 1 und 49,2 g (0,6 mol) Natriumacetat 87,5 g.
Ausbeute : 25,9 % der Theorie
$^1$H-NMR ($CDCl_3$) : $\delta$ = 1,15 (tr, 2H, $CH_3$); 2,15 (br, s, NH); 2,6 (s, 3H, $CH_3$); 4,1 (q, 2H, $CH_2$); 5,8 (s, 1H, CH); 7,1-8,0 (9H, Aromaten-H) ppm.

Beispiel 10

4-(4-Fluorphenyl)-6-methyl-2-phenyl-pyrimidin-5-carbonsäureethylester

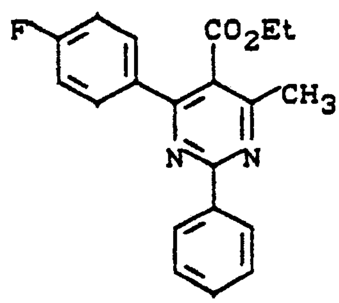

Analog Beispiel 3 erhält man aus 40 g (0,12 mol) Beispiel 9 und 11,8 g (0,12 mol) Chromtrioxid 34,9 g.
Ausbeute: 87,9 % der Theorie
$^1$H-NMR ($CDCl_3$) : $\delta$ = 1,15 (tr, 3H, $CH_3$); 2,7, (s, 3H, $CH_3$); 4,25 (q, 2H, $CH_2$); 7,1-8,6 (9H, Aromaten-H) ppm.

Beispiel 11

4-(4-Fluorphenyl)-5-hydroxymethyl-6-methyl-2-phenyl-pyrimidin

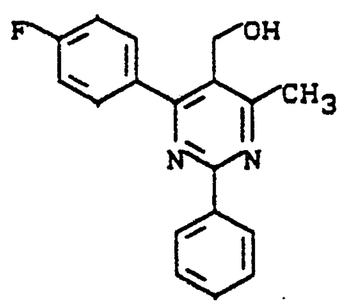

Analog Beispiel 4 erhält man aus 30 g (0,09 mol) Beispiel 10 23,1 g.
Ausbeute: 87,3 % der Theorie
$^1$H-NMR ($CDCl_3$) : $\delta$ = 2,8 (s, 3H, $CH_3$); 4,5 (br, OH); 4,65 (s, 2H, $CH_2$); 7,1-8,5 (9H, Aromaten-H) ppm.

Beispiel 12

4-(4-Fluorphenyl)-6-methyl-2-phenyl-pyrimidin-5-carbaldehyd

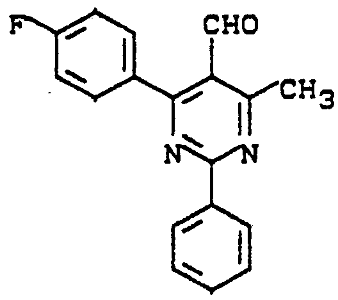

Analog Beispiel 5 erhält man aus 12,9 g (44 mmol) Beispiel 11 und 14,2 g (66 mmol) Pyrimidiniumchlorochromat 11,9 g.
Ausbeute: 93 % der Theorie
$^1$H-NMR ($CDCl_3$) : $\delta$ = 2,9 (s, 3H, $CH_3$); 7,2-8,7 (9H, Aromaten-H); 10,1 (s, 1H, CHO)ppm.

Beispiel 13

E-3-[4-(4-Fluorphenyl)-6-methyl-2-phenyl-pyrimid-5-yl]-prop-2-enal

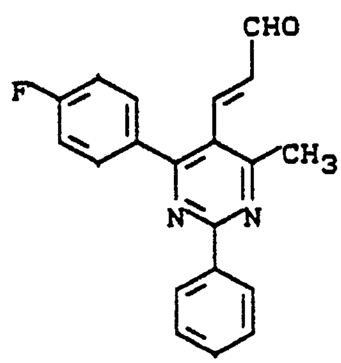

Analog Beispiel 6 erhält man aus 8,0 g (27 mmol) Beispiel 12, 0,8 g (33,6 mmol) Natriumhydrid und 8,0 g (32,4 mmol) [2-(Cyclohexylamino)vinyl]phosphonsäurediethylester 5,9 g.
Ausbeute: 68,7 % der Theorie
$^1$H-NMR ($CDCl_3$) : $\delta$ = 2,8 (s, 3H, $CH_3$); 6,45 (dd, 1H, CHCHO; 7,1-8,6 (m, 1OH, Aromaten-H, CH); 9,65 (d, 1H, CHO) ppm.

Beispiel 14

Methyl-(E)-7-[4-(4-fluorphenyl)-6-methyl-2-phenyl-pyrimid-5-yl]-5-hydroxy-3-oxo-hept-6-enoat

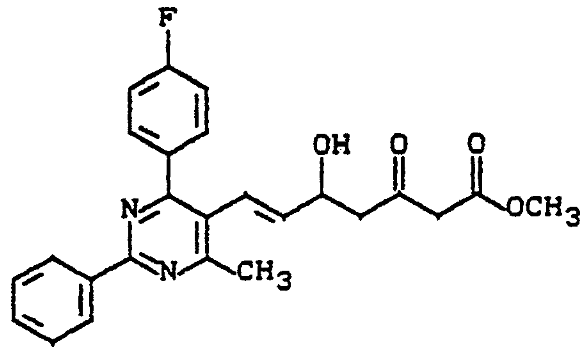

Analog Beispiel 7 erhält man aus 3,4 g (10,7 mmol) Beispiel 13 5,18 g Rohprodukt. Rohausbeute: 100 %
$^1$H-NMR ($CDCl_3$) : $\delta$ = 2,65 (s, 3H, $CH_3$); 2,7 (d, 2H, $CH_2\overset{\overset{O}{\|}}{C}$ ); 2,9 (br, 1H, OH); 3,5 (s, 3H, $CH_3$); 3,75 (s, 3H, $OCH_3$); 4,7 (m, 1H, CHOH); 5,65 (dd, 1H, CH-CHOH); 6,65 (d, 1H, CH Ar); 7,1-8,6 (m, 9H, Aromaten-H) ppm.

## Beispiel 15

Methyl-erythro-(E)-3,5-dihydroxy-7-[4-(4-fluorphenyl)-6-methyl-2-phenyl-pyrimid-5-yl]-hept-6-enoat

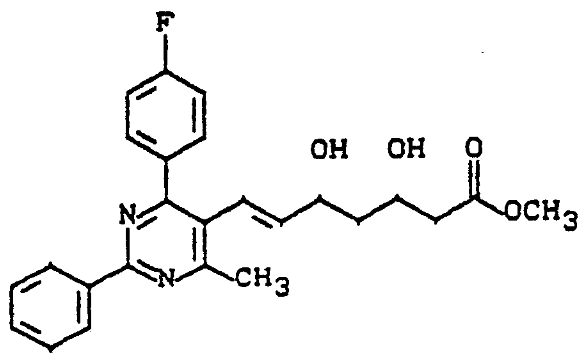

Analog Beispiel 8 erhält man aus 8,26 g (16 mmol) Beispiel 14 4,03 g.
Ausbeute: 57,7 % der Theorie
$^1$H-NMR ($CDCl_3$) : $\delta$ = 1,6 (m, 2H, $CH_2$); 2,5 (m 2H, $CH_2C=O$); 2,7 (s, 3H, $CH_3$); 3,7 (s, 3H, $CH_3O$); 4,2 (m, 1H, CHOH); 4,5 (m, 1H, CHOH); 5,7 (dd, 1H, CH-CHOH); 6,6 (d, 1H, CH-Ar); 7,0-8,6 (m, 9H, Aromaten-H) ppm.

## Beispiel 16

(E/Z)-4-Ethoxycarbonyl-5-(4-fluorphenyl)-2-methyl-pent-4-en-3-on

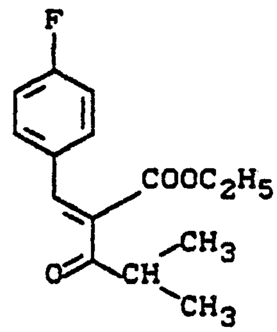

62 g (0,5 mol) 4-Fluorbenzaldehyd und 79 g (0,5 mol) Isobutyrylessigsäureethylester werden in 300 ml trockenem Isopropanol vorgelegt und mit einem Gemisch aus 2,81 ml (28 mmol) Piperidin und 1,66 ml (29 mmol) Essigsäure in 40 ml Isopropanol versetzt. Man läßt 48 h bei Raumtemperatur rühren, engt im Vakuum ein und destilliert den Rückstand im Hochvakuum.
Kp 0,5 mm; 127° C
Ausbeute: 108,7 g (82,3 % der Theorie).

Beispiel 17

1,4-Dihydro-4-(4-fluorphenyl)-6-isopropyl-2-phenyl-pyrimidin-5-carbonsäureethylester

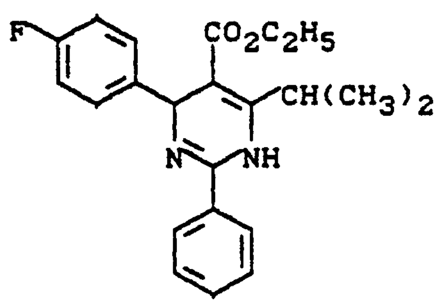

Analog Beispiel 2 erhält man aus 59,5 g (0,3 mol) Benzamidinhydrochlorid, 79,2 g (0,3 mol) Beispiel 16 und 28,7 g (0,35 mol) Natriumacetat in 500 ml Ethanol 39 g. Ausbeute: 35,5 % der Theorie
$^1$H-NMR ($CDCl_3$) : δ = 1,2 (m, 9H, $CH_3$); 2,8 (m, 1 H, CH); 4,1 (q, 2H, $CH_2$); 4,2 (br, 1H, NH); 5,75 (s, 1H, CHAr); 6,9-8,1 (m, 9H, Aromaten-H) ppm.

Beispiel 18

4-(4-Fluorphenyl)-6-isopropyl-2-phenyl-pyrimidin-5-carbonsäureethylester

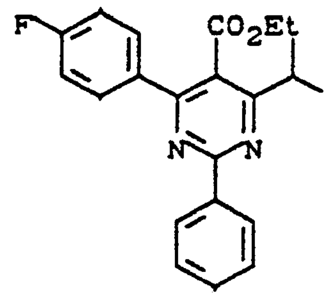

Analog Beispiel 3 erhält man aus 39,0 g (0,11 mol) Beispiel 17 20,6 g.
Ausbeute: 56,6 % der Theorie
$^1$H-NMR ($CDCl_3$) : δ = 1,15 (tr, 3H, $CH_3$); 1,4 (d, 6H, $CH_3$); 3,3 (m, 1H, CH); 4,2 (q, 2H, $CH_2$); 7,0-8,6 (m, 9H, Aromaten-H) ppm.

Beispiel 19

4-(4-Fluorphenyl)-5-hydroxymethyl-6-isopropyl-2-phenyl-pyrimidin

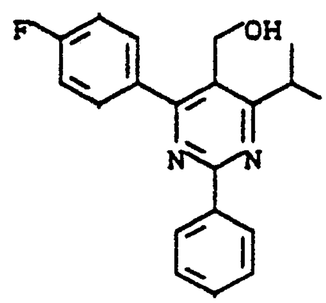

Analog Beispiel 4 erhält man aus 20,0 g (55 mmol) Beispiel 18 16,7 g der Titelverbindung.
Ausbeute: 94 % der Theorie
$^1$H-NMR ($CDCl_3$) : δ = 1,4 (d, 6H, $CH_3$); 1,7 (br, 1H, OH) 3,55 (m, 1H, CH); 4,7 (s, 2H, $CH_2$); 7,1-8,7 (m, 9H, Aromaten-H) ppm.

Beispiel 20

4-(4-Fluorphenyl)-6-isopropyl-2-phenyl-pyrimidin-5-carbaldehyd

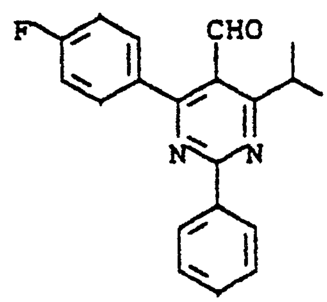

Analog Beispiel 5 erhält man aus 16,3 g (50 mmol) Beispiel 19 7,9 g der Titelverbindung.

Ausbeute: 49,4 % der Theorie
$^1$H-NMR ($CDCl_3$) : $\delta$ = 1,4 (d, 6H, $CH_3$); 4,0 (m, 1H, CH); 7,2-8,7 (m, 9H, Aromaten-H), 10,1 (s, 1H, CHO) ppm.

Beispiel 21

(E)-3-[4-(4-Fluorphenyl)-6-isopropyl-2-phenyl-pyrimid-5-yl]-prop-2-enal

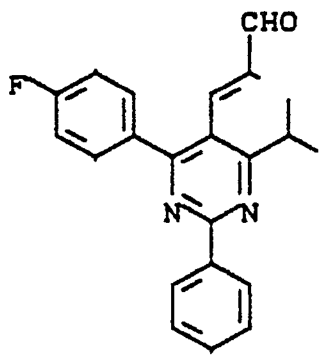

Analog Beispiel 6 erhält man aus 7,9 g (25 mmol) Beispiel 20 6,9 g des Beispiels 21.
Ausbeute: 80 % der Theorie
$^1$H-NMR ($CDCl_3$) : $\delta$ = 1,4 (d, 6H, $CH_3$); 3,4 (m, 1H, CH); 6,3 (dd, 1H, CHCHO); 7,1-8,7 (m, 9H, Aromaten-H); 7,6 (d, 1H, CHAr) ppm.

Beispiel 22

Methyl-(E)-7-[4-(4-fluorphenyl)-6-isopropyl-2-phenyl-pyrimid-5-yl]-5-hydroxy-3-oxo-hept-6-enoat

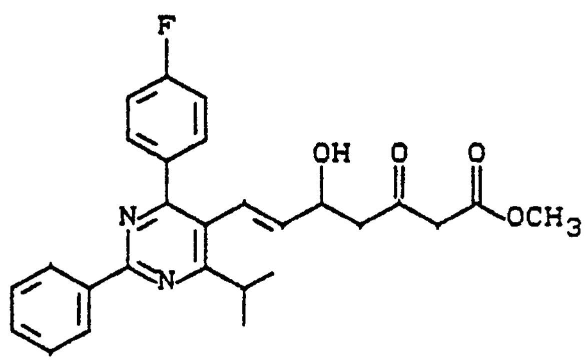

Analog Beispiel 7 erhält man aus 1,9 g (5,5 mmol) Beispiel 21 0,8 g des Beispiels 22.
Ausbeute: 30,2 %
$^1$H-NMR ($CDCl_3$) : $\delta$ = 1,35 (d, 6H, $CH_3$); 2,65 (d, 2H, $CH_2$); 3,4 (m, 1H, CH); 3,5 (s, 2H, $CH_2$); 3,75 (s, 3H, $CH_3O$; 4,65 (m, 1H, CHOH); 5,5 (dd, 1H, CH CHOH); 6,75 (d, 1H, CHAr); 7,1-8,6 (m, 9H, Aromaten-H) ppm.

Beispiel 23

Methyl-erythro-(E)-3,5-dihydroxy-7-[4-(4-fluorphenyl)-6-isopropyl-2-phenyl-pyrimid-5-yl]-hept-6-enoat

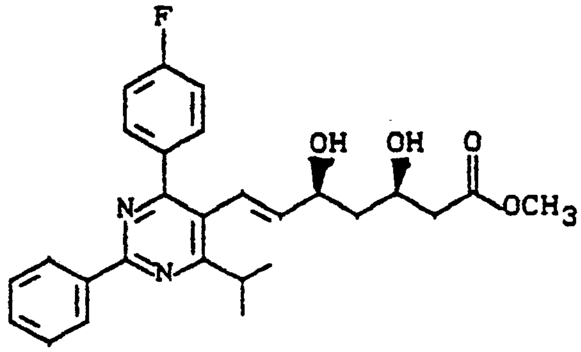

Analog Beispiel 8 erhält man aus 0,8 g (1,75 mmol) Beispiel 22 0,5 g des Beispiels 23.
Ausbeute: 62 %
$^1$H-NMR ($CDCl_3$) : δ = 1,3 (d, 6H, $CH_3$); 1,6 (m, 2H, $CH_2$); 2,5 (m, 2H, $CH_2C=O$); 3,45 (m, 1H, CH); 3,7 (s, 3H, $CH_3O$); 4,2 (m, 1H, CHOH); 4,5 (m, 1H, CHOH); 5,5 (dd, 1H, CH-CHOH); 6,7 (d, 1H, CHAr); 7,1-8,6 (m, 9H, Aromaten-H) ppm.

Anwendungsbeispiele

Beispiel 1

Die Enzymaktivitätsbestimmung wurde modifiziert nach G.C. Ness et al., Archives of Biochemistry and Biophysics 197, 493-499 (1979) durchgeführt. Männliche Ricoratten (Körpergewicht 300-400 g) wurden 11 Tage mit Altrominpulverfutter, dem 40 g Cholestyramin/kg Futter zugesetzt war, behandelt. Nach Dekapitation wurde den Tieren die Leber entnommen und auf Eis gegeben. Die Lebern wurden zerkleinert und im Potter-Elvejem-Homogenisator 3 mal in 3 Volumen 0,1 m Saccharose, 0,05 m KCl, 0,04 m $K_xH_y$ Phosphat, 0.03 m Ethylendiamintetraessigsäure, 0,002 m Dithiothreit (SPE)-Puffer pH 7,2, homogenisiert. Anschließend wurde 15 Minuten bei 15 000* g zentrifugiert und das Sediment verworfen. Der Überstand wurde 75 Minuten bei 100 000 g sedimentiert. Das Pellet wird in 1/4 Volumen SPE-Puffer aufgenommen, nochmals homogenisiert und anschließend erneut 60 Minuten bei 1000 000 g zentrifugiert. Das Pellet wird mit der 5-fachen Mengen ihres Volumens SPE-Puffer aufgenommen, homogenisiert und bei -78° C eingefroren und gelagert (= Enzymlösung).

Zur Testung wurden die Testverbindungen (oder Mevinolin als Referenzsubstanz) in Dimethylformamid unter Zugabe von 5 Vol. -% 1 n NaOH gelöst und mit 10 µl in verschiedenen Konzentrationen in den Enzymtest eingesetzt. Der Test wurde nach 20 Minuten Vorinkubation der Verbindungen mit dem Enzym bei 37° C gestartet. Der Testansatz betrug 0,380 ml und enthielt 4 µMol Glucose-6-Phosphat, 1,1 mg Rinderserumalbumin, 2,1 µMol Dithiothreit, 0,35 µMol NADP, 1 Einheit Glucose-6-Phosphatdehydrogenase 35 µMol $K_xH_y$-Phosphat pH 7,2, 20 µl enzympräparation und 56 nMol 3-Hydroxy-3-methyl-glutaryl Coenzym A (Glutaryl-3-$^{14}$C) 1000 000 dpm.

Man inkubierte 60 Minuten bei 37° C und stoppte die Reaktion durch Zusatz von 300 µl 0,24 m HCl ab. Mach einer Nachinkubation von 60 Minuten bei 37° C wurde der Ansatz zentrifugiert und 600 µl des Überstandes auf eine 0,7 x 4 cm mit Biorex® 5-Chlorid 100-200 mesh (Anionenaustauscher) gefüllte Säule aufgetragen. Es wurde mit 2 ml dest. Wasser nachgewaschen und Durchlauf plus Waschwasser mit 3 ml Aquasol versetzt und im LKB-Scintillationszähler gezählt. $IC_{50}$-Werte wurden durch Auftrag der prozentualen Hemmung gegen die Konzentration der Verbindung im Test durch Intrapolation bestimmt. Zur Bestimmung der relativen inhibitorischen Potenz wurde der $IC_{50}$-Wert der Referenzsubstanz Mevinolin als 1 gesetzt und mit dem simultan bestimmten $IC_{50}$-Wert der Testverbindung verglichen.

Die Wirkstoffe der Beispiele 1 bis 23 zeigen eine im Vergleich zu Mevinolin höhere Wirkung.

Beispiel 2

Die Serum-Cholesterin-senkende Wirkung der erfindungsgemäßen Verbindungen auf die Blutcholesterinwerte von Hunden und Meerschweinchen wurd ein mehrwöchigen Fütterungsexperimenten gefunden. Dazu wurde die zu untersuchende Substanz im Fall der Hundeversuche über einen mehrwöchigen zeitraum einmal täglich i einer Kapsel gesunden Beagle Hund zusammenb mit dem Futter p.o. gegeben. Dem Futter war außerdem während der gesamten Versuchsperiode, d.h. vor, während und nach der Applikationsperiode der zu untersuchenden Susbtanz Colestyramin (4 g/100 g Futter) als Gallensäuresequetrant beigemischt.

Zweimal wöchentlich wurde den Hunden venöses Blut abgenommen und das Serumcholesterin mit einem handelsübilchen Testkit enzymatisch bestimmt. Die Serumcholestrinwerte währen der Applikationsperiode wurden mi den Serumcholesterinwerten vor der Applicationsperiode (Kontrollen) verglichen.

Den Meerschweinchen wurde die Substanz in das Futter gemischt. Nach zwei Wochen wurden den Tieren Blut entnommen und das Serum-Cholesterin bestimmt.

## Ansprüche

1. Substituierte Pyrimidine der allgemeinen Formel

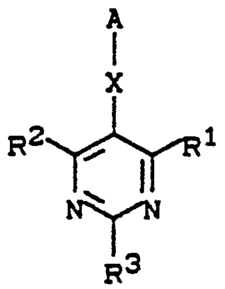

(I)

in welcher

$R^1$ - für Cycloalkyl steht, oder

- für Alkyl steht, das substituiert sein kann durch Halogen, Cyano, Alkoxy, Alkylthio, Alkylsulfonyl, Trifluormethyl, Trifluormethoxy, Trifluormethylthio, Trifluormethylsulfonyl, Alkoxycarbonyl, Acyl oder durch eine Gruppe der Formel - $NR^4R^5$, worin

$R^4$, $R^5$ - gleich oder verschieden sind und Alkyl, Aryl, Aralkyl, Acyl, Alkylsulfonyl oder Arylsulfonyl bedeuten,

oder durch Carbamoyl, Dialkylcarbamoyl, Sulfamoyl, Dialkylsulfamoyl, Heteroaryl, Aryl, Aryloxy, Arylthio, Arylsulfonyl, Aralkoxy, Aralkylthio oder Aralkylsulfonyl, wobei die Heteroaryl- und Arylreste der letztgenannten Substituenten bis zu 3-fach gleich oder ver schieden durch Halogen, Cyano, Trifluormethyl, Trifluormethoxy, Alkyl, Alkoxy, Alkylthio oder Alkylsulfonyl substituiert sein können,

$R^2$ - für Heteroaryl steht, das bis zu 3-fach gleich oder verschieden durch Halogen, Alkyl, Alkoxy, Alkylthio, Alkylsulfonyl, Aryl, Aryloxy, Arylthio, Arylsulfonyl, Trifluormethyl, Trifluormethoxy, Trifluormethylthio, Alkoxycarbonyl oder durch eine Gruppe der Formel -$NR^4R^5$ substituiert sein kann, worin

$R^4$ und $R^5$ die oben angegebene Bedeutung haben, oder

$R^2$ - für Aryl steht, das bis zu 5-fach gleich oderverschieden substituiert sein kann durch Alkyl, Alkoxy, Alkylthio, Alkylsulfonyl, Aryl, Aryloxy, Arylthio, Arylsulfonyl, Aralkyl, Aralkoxy, Aralkylthio, Aralkylsulfonyl, Halogen, Cyano, Nitro, Trifluormethyl, Trifluormethoxy, Trifluormethylthio, Alkoxycarbonyl, Sulfamoyl, Dialkylsulfamoyl, Carbamoyl, Dialkylcarbamoyl oder durch eine Gruppe der Formel -$NR^4R^5$, worin $R^4$,$R^5$ die oben angegebene Bedeutung haben,

$R^3$ - für Wasserstoff oder

- für Cycloalkyl steht, oder

- für Alkyl steht, die substituiert sein können durch Halogen, Cyano, Alkoxy, Alkylthio, Alkylsulfonyl, Trifluormethyl, Trifluormethoxy, Trifluormethylthio, Trifluormethylsulfonyl, Alkoxycarbonyl, Acyl oder durch eine Gruppe der Formel -$NR^4R^5$, worin

$R^4$ und $R^5$ die oben angegebene Bedeutung haben,

oder durch Carbamoyl, Dialkylcarbamoyl, Sulfamoyl, Dialkylsulfamoyl, Heteroaryl, Aryl, Aryloxy, Arylthio, Arylsulfonyl, Aralkoxy, Aralkylthio oder Aralkylsulfonyl, wobei die Heteroaryl- und Arylreste der letztgenannten Substituenten bis zu 3-fach gleich oder verschieden durch Halogen, Cyano, Trifluormethyl, Trifluormethoxy, Alkyl, Alkoxy, Alkylthio oder Alkylsulfonyl substituiert sein können, oder

$R^3$ - für Heteroaryl steht, das bis zu 3-fach gleich oder verschieden durch Halogen, Alkyl, Alkoxy, Alkylthio,

Alkylsulfonyl, Aryl, Aryloxy, Arylthio, Arylsulfonyl, Trifluormethyl, Trifluormethoxy, Trifluormethylthio, Alkoxycarbonyl oder durch eine Gruppe der Formel -$NR^4R^5$ substituiert sein kann, worin

$R^4$ und $R^5$ die oben angegebene Bedeutung haben, oder

$R^3$ - für Aryl steht, das bis zu 5-fach gleich oder verschieden substituiert sein kann durch Alkyl, Alkoxy, Alkylthio, Alkylsulfonyl, Aryl, Aryloxy, Arylthio, Arylsulfonyl, Aralkyl, Aralkoxy, Aralkylthio, Aralkylsulfonyl, Halogen, Cyano, Nitro, Trifluormethyl, Trifluormethoxy, Trifluormethylthio, Alkoxycarbonyl, Sulfamoyl, Dialkylsulfamoyl, Carbamoyl, Dialkylcarbamoyl oder durch eine Gruppe der Formel -$NR^4R^5$, worin

$R^4$ und $R^5$ die oben angegebene Bedeutung haben, oder

$R^3$ - für Alkox, Aryloxy, Aralkox, Alkylthio, Arylthio, Aralkylthio oder für eine Gruppe der Formel $NR^4R^5$ steht, worin

$R^4$ und $R^5$ die oben angegebene Bedeutung haben,

X - für eine Gruppe der Formel -$CH_2$-$CH_2$- oder -CH=CH-steht, und

A - für eine Gruppe der Formel

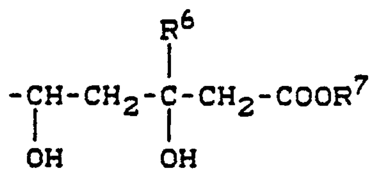

oder

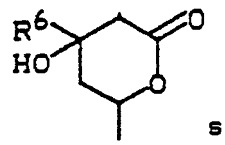

steht, worin

$R^6$ - Wasserstoff oder Alkyl bedeutet und

$R^7$ - Wasserstoff,

- einen Methyl-, Aryl- oder Aralkylrest oder

- ein Kation bedeutet.

2. Substituierte Pyrimidine nach Anspruch 1 wobei

$R^1$ - für Cyclopropyl, Cyclopentyl oder Cyclohexyl steht, oder

- für Niederalkyl steht, das substituiert sein kann durch Fluor, Chlor, Brom, Cyano, Niederalkoxy, Niederalkylthio, Niederalkylsulfonyl, Trifluormethyl, Trifluormethoxy, Trifluormethylsulfonyl, Niederalkoxycarbonyl, Benzoyl, Niederalkylcarbonyl, durch eine Gruppe der Formel-$NR^4R^5$, worin

$R^4$ und $R^5$ gleich oder verschieden sind und Niederalkyl, Phenyl, Benzyl, Acetyl, Benzoyl, Phenylsulfonyl oder Niederalkylsulfonyl bedeuten,

oder durch Pyridyl, Pyrimidyl, Pyrazinyl, Pyridazinyl, Chinolyl, Isochinolyl, Pyrryl, Indolyl, Thienyl, Furyl, Imidazolyl, Oxazolyl, Thiazolyl, Phenyl, Phenoxy, Phenylthio, Phenylsulfonyl, Benzyloxy, Benzylthio, Benzylsulfonyl, Phenylethoxy, Phenylethylthio oder Phenylethyl sulfonyl, wobei die genannten Heteroaryl- und Arylreste bis zu 2-fach gleich oder verschieden durch Fluor, Chlor, Brom, Niederalkyl, Niederalkoxy, Trifluormethyl, oder Trifluormethoxy substituiert sein können,

$R^2$ - für Thienyl, Furyl, Thiazolyl, Isothiazolyl, Oxazolyl, Isoxazolyl, Pyridyl, Pyrimidyl, Pyrazinyl, Pyridazinyl, Indolyl, Isoindolyl, Chinolyl, Isochinolyl, Phthalazinyl, Chinoxalinyl, Chinazolinyl, Cinnolinyl, Benzothiazolyl, Benzoxazolyl oder Benzimidazolyl steht, das bis zu 2-fach gleich oder verschieden substituiert sein kann durch Fluor, Chlor, Brom, Niederalkyl, Niederalkoxy, Phenyl, Phenoxy, Trifluormethyl, Trifluormethoxy oder Niederalkoxycarbonyl, oder

- für Phenyl oder Naphthyl steht, die bis zu 4-fach gleich oder verschieden substituiert sein kann durch Niederalkyl, Niederalkoxy, Niederalkylthio, Niederalkylsulfonyl, Phenyl, Phenyloxy, Phenylthio, Phenylsulfonyl, Benzyl, Benzyloxy, Benzylthio, Benzylsulfonyl, Phenethyl, Phenylethoxy, Phenylethylthio, Phenylethylsulfonyl, Fluor, Chlor, Brom, Cyano, Trifluormethyl, Trifluormethoxy, Trifluormethylthio, Niederalkoxycarbonyl oder durch eine Gruppe der Formel -$NR^4R^5$, wobei $R^4$, $R^5$ die oben angegebene Bedeutung haben,

$R^3$ - für Wasserstoff oder

- für Cyclopropyl, Cyclopentyl oder Cyclohexyl steht oder

- für Niederalkyl steht, das substituiert sein kann durch Fluor, Chlor, Brom, Cyano, Niederalkoxy, Niederalkylthio, Niederalkylsulfonyl, Trifluormethyl, Trifluormethoxy, Trifluormethylsulfonyl, Niederalkoxycarbonyl, Benzoyl, Niederalkylcarbonyl, durch eine Gruppe der Formel -$NR^4R^5$, worin $R^4$, $R^5$ die oben angegebene Bedeutung haben,

oder durch Pyridyl, Pyrimidyl, Pyrazinyl, Pyridazinyl, Chinolyl, Isochinolyl, Pyrrolyl, Indolyl, Thienyl, Furyl, Imidazolyl, Oxazolyl, Thiazolyl, Phenyl, Phenoxy, Phenylthio, Phenylsulfonyl, Benzyloxy, Benzylthio, Benzylsulfonyl, Phenylethoxy, Phenylethylthio oder Phenylethylsulfonyl, wobei die genannten Heteroaryl- und Arylreste bis zu 2-fach gleich oder verschieden durch Fluor, Chlor, Brom, Niederalkyl, Niederalkoxy, Trifluormethyl, oder Trifluormethoxy substituiert sein können, oder

$R^3$ - für Thienyl, Furyl, Thiazolyl,Isothiazolyl, Oxazolyl, Isoxazolyl, Pyridyl, Pyrimidyl, Pyrazinyl, Pyridazinyl, Indolyl, Isoindolyl, Chinolyl, Isochinolyl, Phthalazinyl, Chinoxalinyl, Chinazolinyl, Cinnolinyl, Benzothiazolyl, Benzoxazolyl oder Benzimidazolyl steht, das bis zu 2-fach gleich oder verschieden substituiert sein kann durch Fluor, Chlor, Brom, Niederalkyl, Niederalkoxy, Phenyl, Phenoxy, Trifluormethyl, Trifluormethoxy oder Niederalkoxycarbonyl, oder - für Phenyl oder Naphthyl steht, das bis zu 4-fach gleich oder verschieden substituiert sein kann durch Niederalkyl, Niederalkoxy, Niederalkylthio, Niederalkylsulfonyl, Phenyl, Phenyloxy, Phenylthio, Phenylsulfonyl, Benzyl, Benzyloxy, Benzylthio, Benzylsulfonyl, Phenethyl, Phenylethoxy, Phenylethylthio, Phenylethylsulfonyl, Fluor, Chlor, Brom, Cyano, Trifluormethyl, Trifluormethoxy, Trifluormethylthio, Niederalkoxycarbonyl oder durch eine Gruppe der Formel $-NR^4R^5$, oder

$R^4$ und $R^5$ die oben angegebene Bedeutung haben, oder

$R^3$ - für Alkoxy, Aryloxy, Aralkoxy, Alkylthio, Arylthio, Aralkylthio oder für eine Gruppe derr Formel $NR^4R^5$ steht, worin

$R^4$ und $R^5$ die oben angegebene Bedeutung haben,

X - für eine Gruppe der Formel $-CH_2-CH_2-$ oder $-CH=CH-$steht, und

A - für eine Gruppe der Formel

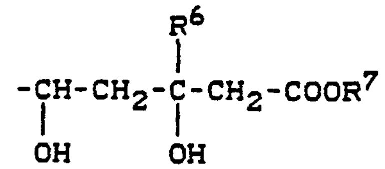 oder 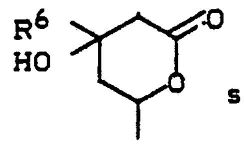 steht, worin

$R^6$ - Wasserstoff oder Niederalkyl bedeutet, und

$R^7$ - einen Alkyl ($C_1$ bis $C_6$), Aryl ($C_6$ bis $C_{12}$) oder Aralkyl ($C_7$ bis $C_{12}$) oder

- ein physiologisch verträgliches Kation bedeutet.

3. Substituierte Pyrimidine nach den Ansprüchen 1 und 2, wobei

$R^1$ - für Cyclopropyl, Cyclopentyl oder Cyclohexyl steht, oder

- für Methyl, Ethyl, Propyl, Isopropyl, Butyl, sec.-Butyl oder tert.Butyl steht, die substituiert sein können durch Fluor, Chlor, Brom, Cyano, Methoxy, Ethoxy, Propoxy, Isopropoxy, Butoxy, sec.Butoxy, tert.Butoxy, Methylthio, Ethylthio, Propylthio, Isopropylthio, Methylsulfonyl, Ethylsulfonyl, Propylsulfonyl, Isopropylsulfonyl, Trifluormethyl, Trifluormethoxy, Methoxycarbonyl, Ethoxycarbonyl, Butoxycarbonyl, Isobutoxycarbonyl, tert.-Butoxycarbonyl, Benzoyl, Acetyl, Pyridyl, Pyrimidyl, Thienyl, Furyl, Phenyl, Phenoxy, Phenylthio, Phenylsulfonyl, Benzyloxy, Benzylthio oder Benzylsulfonyl,

$R^2$ - für Pyridyl, Pyrimidyl, Chinolyl oder Isochinolyl stehen, die durch Fluor, Chlor, Methyl, Methoxy oder Trifluormethyl substituiert sein können, oder

- für Phenyl steht, das bis zu 3-fach gleich oder verschieden substituiert sein kann durch Methyl, Ethyl, Propyl, Isopropyl, Butyl, Isobutyl, tert.-Butyl, Methoxy, Ethoxy, Propoxy, Isopropoxy, Butoxy, Isobutoxy, tert.-Butoxy, Methylthio, Ethylthio, Propylthio, Isopropylthio, Methylsulfonyl, Ethylsulfonyl, Propylsulfonyl, Isopropylsulfonyl, Phenyl, Phenoxy, Benzyl, Benzyloxy, Fluor, Chlor, Brom, Cyano, Trifluormethyl, Trifluormethoxy, Methoxycarbonyl, Ethoxycarbonyl, Propoxycarbonyl, Isopropoxycarbonyl, butoxycarbonyl, Isobutoxy carbonyl oder tert.Butoxycarbonyl,

$R^3$ - für Wasserstoff, Cyclopropyl, Cyclopentyl oder Cyclohexyl steht, oder

- für Methyl, Ethyl, Propyl, Isopropyl, Butyl, Isobutyl, tert.Butyl, Pentyl, Isopentyl, Hexyl oder Isohexyl stehen, die substituiert sein können durch Fluor, Chlor, Brom, Cyano, Methoxy, Ethoxy, Propoxy, Isopropoxy, Butoxy, Isobutoxy, tert.Butoxy, Methylthio, Ethylthio, Propylthio, Isopropylthio, Butylthio, Isobutylthio, tert.Butylthio, Methylsulfonyl, Ethylsulfonyl, Propylsulfonyl, Isopropylsulfonyl, Butylsulfonyl, Isobutylsulfonyl, tert.Butylsulfonyl, Trifluormethyl, Trifluormethoxy, Methoxycarbonyl, Ethoxycarbonyl, Propoxycarbonyl, Isopropoxycarbonyl, Butoxycarbonyl, Isobutoxycarbonyl, tert.Butoxycarbonyl, Benzoyl, Acetyl, Ethylcarbonyl, oder durch eine Gruppe $-NR^4R^5$, wobei

$R^4$ und $R^5$ gleich oder verschieden sind und Methyl, Ethyl, Propyl, Isopropyl, Butyl, Isobutyl, tert.Butyl, Phenyl, Benzyl, Acetyl, Methylsulfonyl, Ethylsulfonyl, Propylsulfonyl, Isopropylsulfonyl oder Phenylsulfonyl bedeuten,

oder durch Pyridyl, Pyrimidyl, Pyrazinyl, Pyridazinyl, Chinolin, Isochinolyl, Thienyl, Furyl, Phenyl, Phenoxy, Phenylthio, Phenylsulfonyl, Benzyloxy, Benzylthio oder Benzylsulfonyl, wobei die genannten Heteroaryl- und Arylreste durch Fluor, Chlor, Methyl, Ethyl, Propyl, Isopropyl, Isobutyl, tert.Butyl, Methoxy, Ethoxy, Propoxy, Isopropoxy, Butoxy, Isobutoxy, tert.-Butoxy, Trifluormethyl oder Trifluormethoxy substituiert sein können, oder

- für Thienyl, Furyl, Pyridyl, Pyrimidyl, Pyrazinyl, Pyridazinyl, Oxazolyl, Isooxazolyl, Imidazolyl, Pyrazolyl, thiazolyl, Isothiazolyl, Chinolyl, Isochinolyl, Benzoxazolyl, Benzimidazolyl oder Benzthiazolyl steht, wobei die genannten Reste durch Fluor, Chlor, Methyl, Ethyl, Propyl, Isopropyl, Butyl, Isobutyl, tert.Butyl, Methoxy, Ethoxy, Propoxy, Isopropoxy, Butoxy, Isobutoxy, tert.Butoxy, Phenyl, Phenoxy, Trifluormethyl, Trifluormethoxy, Methoxycarbonyl, Ethoxycarbonyl, Isopropoxycarbonyl, Propoxycarbonyl, butoxycarbonyl, Isobutoxycarbonyl oder tert.Butoxycarbonyl substituiert sein können, oder

- für Phenyl steht, das bis zu 3-fach gleich oder verschieden durch Methyl, Propyl, Isopropyl, Butyl, Isobutyl, tert.Butyl, Pentyl, Isopentyl, Hexyl, Isohexyl, Methoxy, Ethoxy, Propoxy, Isopropoxy, Butoxy, Isobutoxy, tert.-Butoxy, Methylthio, Ethylthio, Propylthio, Isopropylthio, Butylthio, Isobutylthio, tert.-Butylthio, Methylsulfonyl, Ethylsulfonyl, Propylsulfonyl, Isopropylsulfonyl, Butylsulfonyl, Isobutylsulfonyl, tert.Butylsulfonyl, Phenyl, Phenoxy, Phenylthio, Phenylsulfonyl, Benzyl, Benzyloxy, Benzylthio, Benzylsulfonyl, Fluor, Chlor, Brom, Cyano, Trifluormethyl, Trifluormethoxy, Trifluormethylthio, Methoxycarbonyl, Ethoxycarbonyl, Propoxycarbonyl, Isopropoxycarbonyl, Butoxycarbonyl, Isobutoxycarbonyl, tert.Butoxycarbonyl oder durch eine Gruppe $-NR^4R^5$ substituiert sein können, wobei

$R^4$ und $R^5$ die oben angegebene Bedeutung haben,

- für Alkoxy, Aryloxy, Aralkoxy, Alkylthio, Arylthio, Aralkylthio oder für eine Gruppe der Formel $NR^4R^5$ steht, worin

$R^4$ und $R^5$ die oben angegebene Bedeutung haben,

X - für ein Gruppe der Formel $-CH_2-CH_2-$ oder $-CH=CH-$steht, und

A - für eine Gruppe der Formel

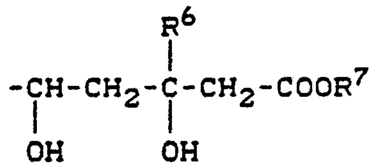 oder 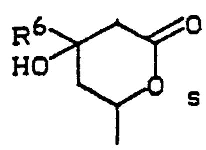 steht, worin

$R^6$ - Wasserstoff, Methyl, Ethyl, Propyl, Isopropyl, Butyl, Isobutyl oder tert.Butyl bedeutet, und

$R^7$ - Wasserstoff, Methyl, Ethyl, Propyl, Isopropyl, Butyl, Isobutyl, tert.Butyl oder Benzyl bedeutet,oder ein Natrium-, Kalium-, Calcium -, oder Magnesium- oder Amoniumion bedeutet.

4. Substituierte Pyrimidine gemäß Anspruch 1 zur therapeutischen Behandlung.

5. Verfahren zur Herstellung von substituierten Pyrimidinen der allgemeinen Formel

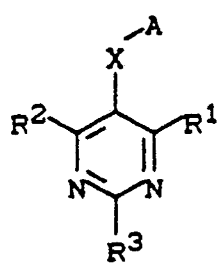 (I)

in welcher

$R^1$ - für Cycloalkyl steht, oder

- für Alkyl steht, das substituiert sein kann durch Halogen, Cyano, Alkoxy, Alkylthio, Alkylsulfonyl, Trifluormethyl, Trifluormethoxy, Trifluormethylthio, Trifluormethylsulfonyl, Alkoxycarbonyl, Acyl, oder durch eine Gruppe der Formel $-NR^4R^5$, worin

$R^4$, $R^5$ - gleich oder verschieden sind und Alkyl, Aryl, Aralkyl, Acyl, Alkylsulfonyl oder Arylsulfonyl bedeuten,

oder durch Carbamoyl, Dialkylcarbamoyl, Sulfamoyl, Dialkylsulfamoyl, Heteroaryl, Aryl, Aryloxy, Arylthio, Arylsulfonyl, Aralkoxy, Aralkylthio oder Aralkylsulfonyl, wobei die Heteroaryl- oder Arylreste der letztgenannten Substituenten bis zu 3-fach gleich oder verschieden durch Halogen, Cyano, Trifluormethyl, Trifluormethoxy, Alkyl, Alkoxy, Alkylthio oder Alkylsulfonyl substituiert sein können,

$R^2$ - für Heteroaryl steht, das bis zu 3-fach gleich oder verschieden durch Halogen, Alkyl, Alkoxy, Alkylthio, Alkylsulfonyl, Aryl, Aryloxy, Arylthio, Arylsulfonyl, Trifluormethyl, Trifluormethoxy, Trifluormethylthio, Alkoxycarbonyl oder durch eine Gruppe der Formel $-NR^4R^5$ substituiert sein kann, worin

$R^4$ und $R^5$ die oben angegebene Bedeutung haben, oder

$R^2$ - für Aryl steht, das bis zu 5-fach gleich oder verschieden substituiert sein kann durch Alkyl, Alkoxy, Alkylthio, Alkylsulfonyl, Aryl, Aryloxy, Arylthio, Arylsulfonyl, Aralkyl, Aralkoxy, Aralkylthio, Aralkylsulfonyl, Halogen, Cyano, Nitro, Trifluormethyl, Trifluormethoxy, Trifluormethylthio, Alkoxycarbonyl, Sulfamoyl, Dialkylsulfamoyl, Carbamoyl, Dialkylcarbamoyl oder durch eine Gruppe der Formel $-NR^4R^5$, worin
$R^4$ und $R^5$ die oben angegebene Bedeutung haben,
$R^3$ - für Wasserstoff oder
- für Cycloalkyl steht, oder
- für Alkyl steht, das substituiert sein kann durch Halogen, Cyano, Alkoxy, Alkylthio, Alkylsulfonyl, Trifluormethyl, Trifluormethoxy, Trifluormethylthio, Trifluormethylsulfonyl, Alkoxycarbonyl, Acyl oder durch eine Gruppe der Formel $-NR^4R^5$, worin
$R^4$, $R^5$ die oben angegebene Bedeutung haben,
oder durch Carbamoyl, Dialkylcarbamoyl, Sulfamoyl, Dialkylsulfamoyl, Heteroaryl, Aryl, Aryloxy, Arylthio, Arylsulfonyl, Aralkoxy, Aralkylthio oder Aralkylsulfonyl, wobei die Heteroaryl- oder Arylreste der letztgenannten Substituenten bis zu 3-fach gleich oder ver schieden durch Halogen, Cyano, Trifluormethyl, Trifluormethoxy, Alkyl, Alkoxy, Alkylthio oder Alkylsulfonyl substituiert sein können, oder
$R^3$ -für Heteroaryl steht, das bis zu 3-fach gleich oder verschieden durch Halogen, Alkyl, Alkoxy, Alkylthio, Alkylsulfonyl, Aryl, Aryloxy, Arylthio, Arylsulfonyl, Trifluormethyl, Trifluormethoxy, Trifluormethylthio, Alkoxycarbonyl oder durch eine Gruppe der Formel $-NR^4R^5$ substituiert sein kann, worin
$R^4$ und $R^5$ die oben angegebene Bedeutung haben, oder
$R^3$ - für Aryl steht, das bis zu 5-fach gleich oder verschieden substituiert sein kann durch Alkyl, Alkoxy, Alkylthio, Alkylsulfonyl, Aryl, Aryloxy, Arylthio, Arylsulfonyl, Aralkyl, Aralkoxy, Aralkylthio, Aralkylsulfonyl, Halogen, Cyano, Nitro, Trifluormethyl, Trifluormethoxy, Trifluormethylthio, Alkoxycarbonyl, Sulfamoyl, Dialkylsulfamoyl, Carbamoyl, Dialkylcarbamoyl oder durch eine Gruppe der Formel $-NR^4R^5$, worin
$R^4$ und $R^5$ die oben angegebene Bedeutung haben oder
$R^3$ - für Alkoxy, Aryloxy, Aralkoxy, Alkylthio, Arylthio, Aralkylthio oder für eine Gruppe der Formel $NR^4R^5$ steht, worin $R^4$ $R^5$ die oben angegebene Bedeutung haben,
X - für eine Gruppe der Formel $-CH_2-CH_2$ oder $-CH=CH-$steht, und
A - für eine Gruppe der Formel

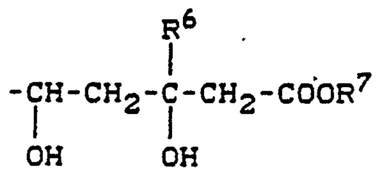

oder

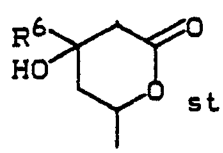

steht, worin

$R^6$ - Wasserstoff oder Alkyl bedeutet und
$R^7$ - Wasserstoff,
- einen Alkyl-, Aryl- oder Aralkylrest oder
- ein Kation bedeutet,
dadurch gekennzeichnet, daß man
Ketone der allgemeinen Formel (VIII)

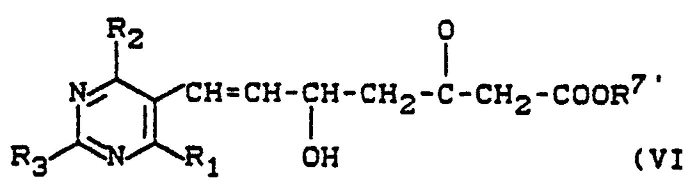

(VIII)

in welcher
$R^1$, $R^2$ und $R^3$ die oben angegebene Bedeutung haben, und
$R^{7'}$ - für Alkyl, Aryl oder Aralkyl steht,
reduziert,
im Fall der Herstellung der Säuren die Ester verseift,
im Fall der Herstellung der Lactone die Carbonsäuren cyclisiert,
im Fall der Herstellung der Salze entweder die Ester oder die Lactone verseift,
im Fall der Herstellung der Ethylenverbindungen ($X = -CH_2-CH_2-$) die Ethenverbindungen ($X = -CH=CH-$)

nach üblichen Methoden hydriert,
und gegebenenfalls Isomeren trennt.

6. Verfahren nach Anspruch 5, dadurch gekennzeichnet das man die Reduktion im Temperaturbereich von -80° C bis +30° C durchführt.

7. Arzneimittel, enthaltend substituierte Pyrimidine gemäß Anspruch 1.

8. Arzneimittel nach Anspruch 7, enthaltend 0,5 -98 Gew.-% an substituierten Pyrimidinen bezogen auf die Zubereitung.

9. Verwendung von substituierten Pyrimidinen gemäß Anspruch 1 zur Behandlung von Krankheiten.

10. Verwendung nach Anspruch 9 zur Behandlung von Hyperlipoproteinämie, Lipoproteinämie oder Atherosklerose.

11. Verwendung von substituierten Pyrimidinen gemäß Anspruch 1 zur Herstellung von Arzneimitteln.

12. Aldehyde der allgemeinen Formel

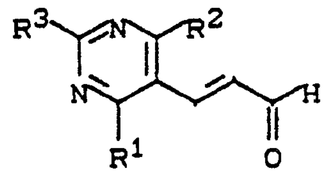

(IX)

in welcher
$R^1$ - für Cycloalkyl steht, oder
- für Alkyl steht, das substituiert sein kann durch Halogen, Cyano, Alkoxy, Alkylthio, Alkylsulfonyl, Trifluormethyl, Trifluormethoxy, Trifluormethylthio, Trifluormethylsulfonyl, Alkoxycarbonyl, Acyl oder durch eine Gruppe der Formel $-NR^4R^5$, worin
$R^4$, $R^5$ -gleich oder verschieden sind und Alkyl, Aryl, Aralkyl, Acyl, Alkylsulfonyl oder Arylsulfonyl bedeuten, oder durch Carbamoyl, Dialkicarbamoyl, Sulfamoyl, Dialkylsulfamoyl, Heteroaryl, Aryl, Aryloxy, Arylthio, Arylsulfonyl, Aralkoxy, Aralkylthio oder Aralkylsulfonyl, wobei die Heteroaryl- und Arylreste der letztgenannten Substituenten bis zu 3-fach gleich oder verschieden durch Halogen, Cyano, Trifluormethyl, Trifluormethoxy, Alkyl, Alkoxy, Alkylthio oder Alkylsulfonyl substituiert sein können,
$R^2$ - für Heteroaryl steht, das bis zu 3-fach gleich oder verschieden durch Halogen, Alkyl, Alkoxy, Alkylthio, Alkylsulfonyl, Aryl, Aryloxy, Arylthio, Arylsulfonyl, Trifluoromethyl, Trifluormethoxy, Trifluormethylthio, Alkoxycarbonyl oder durch eine Gruppe der Form $-NR^4R^5$ substituiert sien kann, worin
$R^4$ und $R^5$ die oben angegebene Bedeutung haben, oder
$R^2$ - für Aryl steht, das bis zu 5-fach gleich oder verschieden substituiert sein kann durch Alkyl, Alkoxy, Alkylthio, Alkylsulfonyl, Aryl, Aryloxy, Arylthio, Arylsulfonyl, Aralkyl, Aralkoxy, Aralkylthio, Aralkylsulfonyl, Halo gen, Cyano, Nitro, Trifluormethyl, Trifluormethoxy, Trifluormethylthio, Alkoxycarbonyl, Sulfamoyl, Dialkylsulfamoyl, Carbamoyl, Dialkylcarbamoyl oder durch eine Gruppe der Formel $-NR^4R^5$, worin
$R^4$ und $R^5$ die oben angegebene Bedeutung haben,
$R^3$ - für Wasserstoff oder
- für Cycloalkyl steht, oder
- für Alkyl steht, das substituiert sein kann durch Halogen, Cyano, Alkoxy, Alkylthio, Alkylsulfonyl, Trifluormethyl, Trifluormethoxy, Trifluormethylthio, Trifluormethylsulfonyl, Alkoxycarbonyl, Acyl oder durch eine Gruppe der Formel $-NR^4R^5$, worin $R^4$, $R^5$ die oben angegebene Bedeutung haben,
oder durch Carbamoyl, Dialkylcarbamoyl, Sulfamoyl, Dialkylsulfamoyl, Heteroaryl, Aryl, Aryloxy, Arylthio, Arylsulfonyl, Aralkoxy, Aralkylthio oder Aralkylsulfonyl, wobei die Heteroaryl- und Arylreste der letztgenannten Substituenten bis zu 3-fach gleich oder verschieden durch Halogen, Cyano, Trifluormethyl, Trifluormethoxy, Alkyl, Alkoxy, Alkylthio oder Alkylsulfonyl substituiert sein können, oder $R^3$ - für Heteroaryl steht, das bis zu 3-fach gleich oder verschieden durch Halogen, Alkyl, Alkoxy, Alkylthio, Alkylsulfonyl, Aryl, Aryloxy, Arylthio, Arylsulfonyl, Trifluormethyl, Trifluormethoxy, Trifluormethylthio, Alkoxycarbonyl oder durch eine Gruppe der Formel $-NR^4R^5$ substituiert sein kann, worin
$R^4$ und $R^5$ die oben angegebene Bedeutung haben, oder
$R^3$ - für Aryl steht, das bis zu 5-fach gleich oder verschieden substituiert sein kann durch Alkyl, Alkoxy, Alkylthio, Alkylsulfonyl, Aryl, Aryloxy, Arylthio, Arylsulfonyl, Aralkyl, Aralkoxy, Aralkylthio, Aralkylsulfonyl, Halogen, Cyano, Nitro, Trifluormethyl, Trifluormethoxy, Trifluormethylthio, Alkoxycarbonyl, Sulfamoyl, Dialkylsulfamoyl, Carbamoyl, Dialkylcarbamoyl oder durch eine Gruppe der Formel $-NR^4R^5$, worin
$R^4$ und $R^5$ die oben angegebene Bedeutung haben, oder
$R^3$ - für Alkoxy, Aryloxy, Aralkoxy, Alkylthio, Arylthio, Aralkylthio oder für eine Gruppe der Formel $NR^4R^5$

steht, worin
$R^4$ und $R^5$ die oben angegebene Bedeutung haben.

13. Verfahren zur Herstellung von Aldehyden der allgemeinen Formel

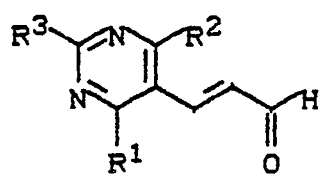

(IX)

in welcher
$R^1$ - für Cycloalkyl steht, oder
- für Alkyl steht, das substituiert sein kann durch Halogen, Cyano, Alkoxy, Alkylthio, Alkylsulfonyl, Trifluormethyl, Trifluormethoxy, Trifluormethylthio, Trifluormethylsulfonyl, Alkoxycarbonyl, Acyl oder durch eine Gruppe der Formel $-NR^4R^5$, worin
$R^4$, $R^5$ -gleich oder verschieden sind und Alkyl, Aryl, Aralkyl, Acyl, Alkylsulfonyl oder Arylsulfonyl bedeuten, oder durch Carbamoyl, Dialkylcarbamoyl, Sulfamoyl, Dialkylsulfamoyl, Heteroaryl, Aryl, Aryloxy, Arylthio, Arylsulfonyl, Aralkoxy, Aralkylthio oder Aralkylsulfonyl, wobei die Heteroaryl- und Arylreste der letztgenannten Substituenten bis zu 3-fach gleich oder
verschieden durch Halogen, Cyano, Trifluormethyl, Trifluormethoxy, Alkyl, Alkoxy, Alkylthio oder Alkylsulfonyl substituiert sein können,
$R^2$ - für Heteroaryl steht, das bis zu 3-fach gleich oder verschieden durch Halogen, Alkyl, Alkoxy, Alkylthio, Alkylsulfonyl, Aryl, Aryloxy, Arylthio, Arylsulfonyl, Trifluormethyl, Trifluormethoxy, Trifluormethylthio, Alkoxycarbonyl oder durch eine Gruppe der Formel $-NR^4R^5$ substituiert sein kann, worin
$R^4$ und $R^5$ die oben angegebene Bedeutung haben, oder
$R^2$ - für Aryl steht, das bis zu 5-fach gleich oder verschieden substituiert sein kann durch Alkyl, Alkoxy, Alkylthio, Alkylsulfonyl, Aryl, Aryloxy, Arylthio, Arylsulfonyl, Aralkyl, Aralkoxy, Aralkylthio, Aralkylsulfonyl, Halogen, Cyano, Nitro, Trifluormethyl, Trifluormethoxy, Trifluormethylthio, Alkoxycarbonyl, Sulfamoyl, Dialkylsulfamoyl, Carbamoyl, Dialkylcarbamoyl oder durch eine Gruppe der Formel $-NR^4R^5$, worin
$R^4$ und $R^5$ die oben angegebene Bedeutung haben,
$R^3$ - für Wasserstoff oder
- für Cycloalkyl steht, oder
- für Alkyl steht, das substituiert sein kann durch Halogen, Cyano, Alkoxy, Alkylthio, Alkylsulfonyl, Trifluormethyl, Trifluormethoxy, Trifluormethylthio, Trifluormethylsulfonyl, Alkoxycarbonyl, Acyl oder durch eine Gruppe der Formel $-NR^4R^5$, worin
$R^4$, $R^5$ die oben angegebene Bedeutung haben,
oder durch Carbamoyl, Dialkylcarbamoyl, Sulfamoyl, Dialkylsulfamoyl, Heteroaryl, Aryl, Aryloxy, Arylthio, Arylsulfonyl, Aralkoxy, Aralkylthio oder Aralkylsulfonyl, wobei die Heteroaryl-und Arylreste der letztgenannten Substituenten bis zu 3-fach gleich oder verschieden durch Halogen, Cyano, Trifluormethyl, Trifluormethoxy, Alkyl, Alkoxy, Alkylthio oder Alkylsulfonyl substituiert sein können, oder
$R^3$ - für Heteroaryl steht, die bis zu 3-fach gleich oder verschieden durch Halogen, Alkyl, Alkoxy, Alk ylthio, Alkylsulfonyl, Aryl, Aryloxy, Arylthio, Arylsulfonyl, Trifluormethyl, Trifluormethoxy, Trifluormethylthio, Alkoxycarbonyl oder durch eine Gruppe der Formel $-NR^4R^5$ substituiert sein können,
worin
$R^4$ und $R^5$ die oben angegebene Bedeutung haben, oder
$R^3$ - für Aryl steht, das bis zu 5-fach gleich oder verschieden substituiert sein kann durch Alkyl, Alkoxy, Alkylthio, Alkylsulfonyl, Aryl, Aryloxy, Ary thio, Arylsulfonyl, Aralkyl, Aralkoxy, Aralkylthi Aralkylsulfonyl, Halogen, Cyano, Nitro, Trifluorm thyl, Trifluormethoxy, Trifluormethylthio, Alkoxy carbonyl, Sulfamoyl, Dialkylsulfamoyl, Carbamoyl, Dialkylcarbamoyl oder durch eine Gruppe der Forme $-NR^4R^5$, worin
$R^4$ und $R^5$ die oben angegebene Bedeutung haben,
dadurch gekennzeichnet, daß man Pyrimidine der allgemeinen Formel

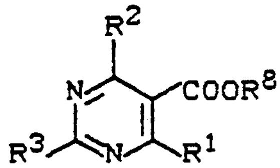

in der $R^1$, $R^2$ und $R^3$ die oben genannte Bedeutung haben und $R^8$ Alkyl ($C_1$ bis $C_6$), Aryl ($C_6$ bis $C_{10}$) oder Aralkyl ($C_7$ bis $C_{10}$) bedeutet, im Temperaturbereich von -70°C bis +100°C in Hydroxymethylenverbindungen der Formel

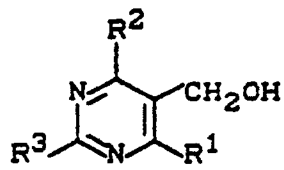

worin $R^1$, $R^2$ und $R^3$ die oben genannte Bedeutung haben, reduziert, anschließend zu den entsprechenden Aldehyden der Formel

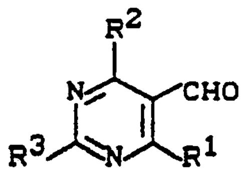

worin $R^1$, $R^2$ und $R^3$ die oben genannte Bedeutung haben, oxidiert und diese mit Dialkyl ($C_1$ bis $C_4$) aminovinylphosphonaten in Anwesenheit von Natriumhydrid in inerten Lösungsmitteln im Temperaturbereich von -20°C bis +40°C umsetzt.